# EUROPEAN PATENT SPECIFICATION

(11) **EP 3 996 790 B1**
(45) Date of publication and mention of the grant of the patent: **18.02.2026**
(21) Application number: 20736970.3
(22) Date of filing: 02.07.2020
(51) Int. Cl.: A61M 25/06

(54) **HYBRID SAFETY SPRING CLIP FOR SHARP MEDICAL DEVICES AND RELATED METHODS**
HYBRIDE SICHERHEITSFEDERKLEMME FÜR SPITZE ODER SCHARFE MEDIZINPRODUKTE UND ZUGEHÖRIGE VERFAHREN
BRIDE DE RESSORT DE SÉCURITÉ HYBRIDE POUR DISPOSITIFS MÉDICAUX POINTUS ET PROCÉDÉS ASSOCIÉS

(30) Priority: 08.07.2019 US 201962871324 P
(43) Date of publication of application: 18.05.2022
(73) Proprietor: B. Braun Melsungen AG, 34212 Melsungen (DE)
(72) Inventor: WOEHR, Kevin, 34587 Felsberg (DE)
(74) Representative: Kinkeldey, Daniela
(86) International application number: PCT/EP2020/068654
(87) International publication number: WO 2021/004880

(56) References cited:
- EP-A1- 2 016 963
- WO-A1-2013/014639
- WO-A1-2014/107133
- WO-A1-2018/186966
- US-A1- 2004 243 061
- US-A1- 2010 222 746

## Description

### FIELD OF ART

The current devices, systems and methods (no methods are claimed) relate to safety spring clips, needle guards, or needle protective devices for use with a needle or an intravenous ("IV") catheter to protect a user from inadvertent needle sticks. In particular, the devices, systems, and methods (no methods are claimed) relate to needle guards made of a combination of metal and plastic to aid in cost-efficient manufacturing.

### BACKGROUND

IV Catheters are commonly used for a variety of infusion therapies. For example, catheters are used for infusing fluids, such as normal saline solution, various medicaments, and total parenteral nutrition, into a patient, withdrawing blood from a patient, or monitoring various parameters of the patient's vascular system. Internal features of IV catheters as conventionally understood can be understood from information regarding various aspects of catheter assemblies and components thereof as discussed in PCT patent applications PCT/EP2016/069619 and PCT/EP2016/069643. There are other sharp medical devices which have hollow bore needles or pins (trocars) for which the needle guards described herein can be used to prevent an inadvertent needle stick.

In order to place a catheter into a vein, cannulation and catheterization can be done with the IV catheter. Generally, the IV catheter assembly can have at least two mated components in a catheter hub with a catheter tube and a cannula hub with a needle or cannula. The needle can extend through the catheter hub and the catheter tube, and the distal tip can extend distally from the catheter tube to allow for cannulation. The needle can be used for insertion and positioning into the patient's vein.

After placement of the catheter tube into the vasculature of the patient, the needle is withdrawn. It is important to prevent accidental needle stick after withdrawing of the needle from the catheter tube and catheter hub. In order to achieve this, needle guards or tip protectors are used to shield the needle tip from accidental needle stick.

The prior art contains a number of needle guards or tip protectors. For example, U.S. Pat. No. 6,616,630 discloses a number of needle guards of varying shapes and structures. Prior art needle guards can be completely passive, not requiring any additional steps to activate the needle guard, or can require an extra pull to remove the needle guard from the catheter hub. Some needle guards or tip protectors require active steps following venipuncture to cover the needle tip, such as depressing a button to release a spring to then retract the needle. US 2004/243061 discloses a safety needle and catheter assembly, and WO 2018/186966 discloses an intravenous catheter assembly with safety clip.

### SUMMARY

The invention is determined by a needle guard according to claim 1. The needle guard comprises a proximal wall having a distally facing surface, a proximally facing surface, and a perimeter defining a through opening, the proximal wall can be made of a first material; a first arm extending distally from the distally facing surface of the proximal wall, the first arm comprising an arm section and a distal wall; a second arm extending distally from the distally facing surface of the proximal wall, the second arm comprising an arm section and a distal wall; wherein one of the first arm and the second arm can be made of a second material different from the first material of the proximal wall. Said differently, the first arm, the second arm, or both the first arm and the second arm can be made of a second material different from the first material of the proximal wall.

For example, the proximal wall can be made from a thermoplastic material and the first resilient arm, the second arm, or both first and second resilient arms made from a metal material. In an example, the first resilient arm, the second arm, or both first and second resilient arms have metal biasing plates placed in contact with exterior surfaces thereof or have metal inserts co-molded or insert molded with the first and second arms, which can be integrally molded with the proximal wall.

The first arm and the second arm of the needle guard can be made of the same second material. The first material can be plastic and the second material can be metal, or the reverse.

The needle guard includes an adaptor attached to the proximal wall.

The adaptor comprises first and second engagement tabs, each engagement tab comprising a locating protrusion for contacting a catheter hub.

The engagement tab can extend from a side surface of an adaptor body of the adapter and can comprise a bend such that the engagement tab extends in a proximal direction or a distal direction.

The engagement tab comprises a locating projection projecting from a surface of the engagement tab.

The adaptor comprises two or more engagement tabs.

The at least two of the two or more engagement tabs can be diametrically opposed around an adaptor body.

The adaptor can have an adaptor body. The body can have engagement tabs extending therefrom with each engagement tab comprising a free end. The body can have a through opening, a cut-out or both a through opening and a cut-out. In some examples, there can be two or more cut-outs and the through opening.

The engagement tabs can extend from the adaptor body and can have free ends that extend in the distal direction or the proximal direction.

The adaptor body of the adaptor can be positioned on a distally facing surface side of the proximal wall of the needle guard. Two or more engagement tabs can extend from the adaptor body with each comprising a free end that can extend in a proximal direction or a distal direction.

The adaptor body of the adaptor can be positioned on a proximally facing surface side of the proximal wall of the needle guard. Two or more engagement tabs can extend from the adaptor body with each comprising a free end that can extend in a proximal direction or a distal direction.

The adaptor body of the adaptor that is positioned distal of the proximal wall of the needle guard can have one or more cut-outs and a through opening for accommodating a needle.

In some examples, the adaptor body can have two cut-outs for accommodating the two arms of the needle guard. The two arms include a first arm and a second arm.

The needle guard comprises a proximal wall, a first arm and a second arm and wherein the first and second arms extend from the proximal wall. The proximal wall can be made from a first material and either or both the first and second arms are made from a second material, which is different from the first material.

The first material can be plastic and the second material can be metallic. The metallic can be insert-molded or co-molded into both the first arm and the second mar, which can also be formed from a plastic material. In some examples, the metallic material can be a biasing plate and the biasing plate can be placed against an exterior of a plastic arm.

The adaptor body of the adaptor can have a curved or arc outer perimeter and a generally straight edge. Where a metallic arm, such as a biasing plate can located closer to the generally straight edge than the curved outer perimeter.

In some examples, there can be two metallic biasing plates and each metallic biasing plate is in contact with either a first plastic arm or a second plastic arm.

The invention is thus defined by a needle guard for a sharp medical device, the needle guard comprising a proximal wall having a distally facing surface, a proximally facing surface, a side surface, and a perimeter defining a through opening; a first arm and a second arm extending distally of the proximal wall; an adaptor at least one of located between the first arm and the second arm and attached to the proximal wall, the adaptor having a first engagement tab and a second engagement tab extending from an adaptor body, each of the first engagement tab and the second engagement tab comprising a locating protrusion for contacting an interior surface of a hub or a housing.

A similar needle guard as described above further comprising a wall guard covering a portion of the first resilient arm is described below but not claimed.

The first engagement tab can extend from a side surface of the adapter body and can comprise a bend such that the engagement tab extends in a proximal or a distal direction from the bend.

The first resilient arm of the needle guard can comprise a tooth to engage with the wall guard.

The needle guard can be located inside an interior of a catheter hub.

The needle guard can be located inside a housing, which is removably connected to the catheter hub.

The needle guard can be mounted on a hypodermic needle and configured to block the needle tip of the needle from unintended needle stick.

The needle guard can have a surface located to a side of the needle shaft and the surface can be movable distally of the needle tip of the needle in an activated position to block the needle tip from unintended needle stick.

The needle guard can comprise an insert having a bore aligned with the through opening of the proximal wall.

Also described but not claimed is a method of manufacturing a needle guard comprising stamping a material sheet to form a central portion and a first pair of opposed strips of material extending from the central portion, the central portion comprising an opening; covering a portion of at least one strip of material with a plastic guard; bending each of the first pair of opposed strips at a first location on the strips and at a second location on the strips in order to form a first resilient arm and a second resilient arm; bending each of the second pair of opposed strips at a first location on the strips to form a first engagement tab and a second engagement tab; wherein the first resilient arm, the second resilient arm, the first engagement tab and the second engagement tab are equally spaced around the central portion.

Broadly speaking, a hybrid clip disclosed herein can comprise a guard made from a plastic material and an adaptor made from a metal material attached to the guard.

The guard can comprise a plastic arm and the adaptor can comprise an engagement tab. The engagement tab can engage the guard.

The needle guard may be sized and shaped to fit in an interior of a catheter hub. The catheter hub can have standard hub dimensions, such as a Luer opening or female Luer at the proximal opening. Optionally, the needle guard, which may alternatively be referred to as a safety spring clip or a needle protective device, may be used with a needle or trocar without a catheter hub. The needle guard may have a proximal wall and at least one needle guard arm, or arm for short.

In some embodiments, the proximal wall may be cylindrical, or disc-like, in shape. The proximal wall may be defined by a cylindrical side surface, a proximally facing wall surface, and an opposed distally facing wall surface. The proximal wall may be sized and shaped to correspond with an interior of a catheter hub. Alternative shapes of the proximal wall may provide for a different cross-sectional shape from the interior of the catheter hub while fitting within the interior of the catheter hub. For example, the proximal wall can be multi-sided shapes and contacting the interior at spaced apart parts, can be oval or elliptical, can be square, can be rectangular, etc.

As used herein and within the medical device industry, the term distal end connotes the end further away from the practitioner and the proximal end is opposite the distal end or closer to the practitioner.

The proximal wall has a perimeter defining an opening. The opening can be sized and shaped to allow a needle or cannula to pass through the proximal wall. If the needle incorporates a crimp, a sleeve, or a material buildup, which can generally be referred to as a change in profile, the diameter of the opening can be smaller than the change in profile so that there is an interference between the opening, or the perimeter of the opening, and the change in profile. This configuration allows the needle to retract in the proximal direction and the change in profile engaging the perimeter on the side of the distally facing surface of the proximal wall during the retraction.

As understood in embodiments of IV catheter assemblies, such as shown in PCT/EP2016/069619, published as WO/2017/029361, the needle, which is attached to a needle hub, can be inserted through the opening of the proximal wall such that it can protrude from a distal opening of a catheter tube when the needle guard is assembled in an IV catheter assembly in a ready to use position.

The needle can have a change in profile located proximally of the needle tip. Relative to the catheter hub in a ready to use position, the change in profile can be distal of the catheter hub. The change in profile can be a crimp or a bulge that has a greater width or diameter than a diameter of the opening as previously described. As such, when the needle is withdrawn or retracted in a proximal direction relative to the needle guard following successful venipuncture, the change in profile catches and engages the perimeter of the opening of the proximal wall. In doing so, the change in profile on the needle prevents the needle from separating from the needle guard when the needle is retracted in the proximal direction, or prevents the needle guard from displacing distally off of the needle.

Two guard arms extend distally of the proximal wall. The two arms can each include sections of different arm widths.

The two arms can each include a first arm section of a first width and a second arm section of a second width, which is greater than the first width. A first end of the first arm section can extend from the proximal wall and a second end of the first arm section can transition to the second arm section.

The two arms can each have a first substantially uniform thickness. In embodiments, one or both of the two arms can have a substantially uniform width instead of the first width and the second width.

In an embodiment, the needle guard described herein can be formed from a plastic material. For example, the needle guard can be formed by plastic injection molding. The two arms of the needle guard can be molded so that they are hinged from the proximal wall and are normally curved or pointed in the direction of the lengthwise axis of the needle guard so that when the arms are spread outwardly away from one another, such as when the needle guard is mounted onto a needle, the two arms bias inwardly towards one another, such as having a restoring force to move towards one another. In some examples, metal components, such as metal inserts, can be co-molded or insert molded with the two arms, and optionally with the proximal wall, to increase the restoring force of the two arms. In still other examples, adapters or attachments can be incorporated with the two arms, such as attached to or glued to the two arms, to increase their restoring force, as further discussed below.

In some embodiments, the first arm section and the second arm section can have a stadium shape cross sectional shape, which can be understood as a geometric shape constructed of a rectangle with semicircles at a pair of opposite corners. However, the cross-sectional shape can embody other geometries, such as rectangular, oval, elliptical, polygonal, irregular, etc. In some embodiments, the first arm section and the second arm section can have a substantially uniform thickness and the first width of the first arm section can transition to the second width of the second arm section with an increasing width tapering section. As such, in embodiments, each of the two arms, which can be resilient, can be understood as having a narrow first arm section extending distally from the proximal wall. The arms can then increase in width in the tapering section as they extend distally from the proximal wall. The second arm section can thereby be a distal portion of the arms that is wider than the first arm section.

As used herein, the arms being resilient is understood to mean being able to spring back into shape. For example, when the two arms are spread apart by the needle, the resilient arms can spring back after retraction of the needle proximally of the one or more distal walls of the needle guard so that the arms can block the needle tip.

The two arms can be circumferentially spaced around the opening on the proximal wall. In some embodiments, the two arms, which can be resilient, can be spaced 180 degrees from each other circumferentially around the opening. In other embodiments, the two arms can be spaced apart at different positions circumferentially from 180 degrees.

Two distal walls can be provided with the needle guard, one at an end of each of the two resilient arms. Each of the distal walls can extend at an angle from the respective second arm section of the two resilient arms. The distal walls may extend towards each other.

The two distal walls can have surfaces with lengths and widths that are sufficient to cover or block a needle tip. The surfaces can be located at or on two different side sections of the needle in a ready to use position and movable distal of the needle in a protective position to block the needle tip from inadvertent needle sticks. The lengths, which can be considered the direction extending from the respective second sections toward the lengthwise axis, or axis X, can be selected so that the two distal walls overlap in a protective position. Alternatively, only one distal wall is incorporated, on one of the two arms, and the other arm can have a free end without a distal wall. Another alternative is a needle guard with only one arm with one distal wall at an end of the one arm.

The distal walls preferably overlap one another along the axis X of the needle guard by utilizing different arm lengths of the two resilient arms and/or angling at least one of the distal walls at an elbow or diagonal section between the distal wall and the resilient arm. In embodiments, one arm can be longer than the other arm. In this way, the different lengths of the two arms allow for staggered, overlapping coverage along the axial direction X, such as when a distal wall is included at an end of each arm.

Alternatively, the two arms can be equal in length such that the distal walls abut one another or the distal walls can be angled from the second arm sections at different angles as they project toward the axis to allow for overlapping.

In fitment with an IV catheter hub of a catheter assembly, the arms can be sufficiently flexible to allow for deflection radially outward such that the needle can extend through the opening and extend distally between the two distal walls of the two arms when the IV catheter assembly is in the ready to use position. In the ready to use position, where the arms are deflected radially outward by the presence of the needle between the two distal walls, the arms preferably have a restoring force acting on the needle.

Upon withdrawal of the needle after successful venipuncture, when the needle is moved proximal of the two distal walls, the arms, which can be resilient and have restoring forces, move back towards the axis X of the needle guard. In this way, the distal walls shield the needle from accidental finger pricks after withdrawal of the needle. The overlapping configuration of the two distal walls can restrict the needle tip from projecting between the two distal walls to re-emerge from the needle tip holding space proximal of the two distal walls.

In some embodiments, the two arms, which include a first arm and a second arm, can each extend substantially parallel to one another. The two arms can each extend perpendicularly from the distally facing wall surface of the proximal wall. The two arms can be singularly formed with the proximal wall and hinged off of the proximal wall.

In other embodiments, the two arms can each extend at an oblique angle relative to the distally facing wall surface of the proximal wall. In embodiments, the two arms can each extend from the distally facing wall surface at an angle ranging from 20 degrees to 89 degrees. More specifically, the two arms can each extend from the distally facing wall surface at an angle ranging from 45 degrees to 80 degrees. The two arms may be angled such that they extend towards each other.

In an embodiment, the two arms of the needle guard are adjacent to one another. In other embodiments, the two arms may intersect and cross over one another. For example, an embodiment where the two resilient arms intersect is shown in PCT/EP2016/069619. The two arms that intersect, can be arms that are made from plastic and the intersection can be at respective smaller cross-sectional arm profiles than larger cross-sectional arm profiles at the distal ends of the two arms.

In other embodiments, more than two guard arms may be used. For example, three or four or more guard arms may be incorporated with a needle guard of the present disclosure. The resilient arms may be equally spaced around the opening at the distally facing wall surface of the proximal wall. Each of the guard arms can be arranged such that distal walls of the guard arms can overlap one another along the axial direction of the needle guard.

Embodiments of the needle guard have a proximal wall and the guard arms may be made of different materials.

In some embodiments, the needle guard may be made from plastic and metal materials. By using both plastic and metal materials, the needle guard may be made in a more economical way by reducing the amount of metal needed.

The metal can be utilized to increase resiliency of the plastic material. The metal can be utilized to prolong storage or shelf life as metal tends not to set over extended periods.

In some embodiments, the proximal wall may be made from a plastic material and the arms may be made from a metal material or be provided with both plastic and metal components, such as forming the arms as a hybrid. Using a metal material may can increase the biasing forces of the arms and increase shelf life as the metal will not set like some plastics might. In the case of some plastics, the plastic may set while being biased in the ready to use position due to a phenomenon called plastic creep. Creep is the tendency of a solid material to move slowly or deform permanently under the influence of mechanical stresses. Thus, including a metal component to the plastic component can maintain the resiliency of the overall hybrid structure even after prolonged storage or after periods of un-used.

Embodiments with the proximal wall made from a plastic material and the arms made at least in part from a metal material may be formed by insert molding. For example, the proximal wall may be insert molded around an end of the resilient arms.

Alternatively, in some embodiments, the proximal wall and the resilient arms may be manufactured separately. The proximal wall may have slots for the insertion of an end of the resilient arms, which can be made from a metal material. In some embodiments, the ends of the resilient arms inserted into the slots at the proximal wall may extend past the proximal side or proximally facing surface of the proximal wall from the distally facing wall surface and then bent, similar to a tab, in order to secure the resilient arms to the proximal wall. Optionally, adhesive or welding may be used to more securely attach the two arms to the proximal wall.

In some embodiments, one of the guard arms may be made from a metal material and another one of the guard arms may be made from a plastic material. In another example, the proximal wall can be made out of metal and have slots, and the arms, made from a plastic material, inserted into the slots in the proximal wall. In embodiments, the proximal end of the plastic arms could include at least one hook feature or projection in order to snap into the slots on the proximal wall and hook themselves fixedly to the proximal wall.

Embodiments of the needle guard formed from plastic material may have the proximal wall and the arms unitarily formed, such as by plastic injection. Alternatively, the proximal wall and the arms may be separately formed and then attached together by plastic welding or adhesives. The arms can be provided with ribs or increased bulk mass extending at least partly along the length of each arm to increase the arm's restoring force.

An adaptor is incorporated with the needle guard. The adaptor has a body or structure for use with the needle guard. In an example, the adaptor can be made from a metal material and can be incorporated to facilitate attachment or securement of the needle guard inside the catheter hub. The adaptor can serve to fix the location of the needle guard in the interior of the catheter hub. For example, the adaptor can assist to secure the needle guard in the catheter hub in the ready to use position and during retraction of the needle following successful venipuncture.

The adaptor can have an adaptor body having a distally facing surface, an opposed proximally facing surface, and a side surface. In some embodiments, the adaptor body can be generally sized and shaped to match the proximal wall of the needle guard but can be differently shaped. The adaptor can be provided with an adaptor body and the proximal wall coupled to one another, as further discussed below.

The adaptor body can have a perimeter defining an opening. The opening on the adaptor can be aligned with the opening of the proximal wall of the needle guard, and the opening of the adaptor can be sized and shaped to allow for the insertion of a needle but sufficiently small to interfere with a change in profile, such as a crimp or an enlarged portion, formed with the needle near the needle tip.

In some embodiments, the adaptor body can be generally cylindrical along an end view. The adaptor body can correspond in shape and size to match the proximal wall of the needle guard. That is, the side surface of the adaptor can have a same diameter as the side surface of the proximal wall of the needle guard.

In other embodiments, only parts or sections of the body of the adaptor can have points that are coincident with the side surface of the proximal wall of the needle guard. For example, a square adaptor body can have four points that lie on the same outer surface profile as the side surface of the proximal wall. In other examples, the four points can be recessed within the outer surface profile of the proximal wall.

At least two engagement tabs extend from the adaptor body. **In** an example, the at least two engagement tabs can extend from the side surface of the adaptor. In an example, there are four engagement tabs. However, there can be fewer than four but at least two or there can be more than four engagement tabs extending from the adaptor body. The engagement tabs can be equally spaced around the side surface or can be randomly spaced around the side surface of the adaptor body.

The engagement tabs may join to the side surface at an angle through a bend or elbow section. That is, the engagement tabs can extend in the proximal direction due to the bend. In some embodiments, the bend may be a 90-degree bend. As such, the engagement tabs can be located at a position radially outward from the adaptor body. In some examples, the engagement tabs are stamped from the same metal blank as the adaptor body and then the bends or elbows are simply formed as by-products in cold-working the tabs to point in the proximal direction.

Each of the engagement tabs has at least one locating protrusion or projection. The locating projection can project from a surface of the engagement tab. In an example, the locating projection projects from the side or surface of the engagement tab that faces the interior of a hub housing, such as facing the interior surface of a catheter hub or a third hub, which is understood to be different from the catheter hub and the needle hub. For example, the third hub can have a housing with an interior and wherein the needle guard can be located inside the interior of the third hub.

The at least one locating protrusion is oriented to contact a surface, such as the interior surface of a hub, such as catheter hub or third hub. Thus, the engagement tab can face radially outward away from the axis X of the needle guard. The locating protrusion may be a hemispherical protrusion or a portion of a sphere on the engagement tab. Alternatively, other geometric shapes could be used, such as a rectangular rib, a trapezoidal or half or partial oval shape. Preferably locating protrusion is smooth to avoid scratching the inside of the catheter hub. The locating protrusion can be arranged to contact an interior of a catheter hub, which can have a standard female Luer or formed as a two-part housing. It can be envisioned that the locating protrusion can be chosen in order to reduce the amount of surface contact and thereby reduce the frictional holding force of the engagement tabs in a catheter hub. In other examples, the adaptor can be omitted and the locating protrusions can be formed directly along the periphery of the side surface of the proximal wall.

The adaptor body may be separately formed and then attached to the proximal wall, such as by welding or bonding. The distally facing surface of the adaptor can contact the proximally facing surface of the proximal wall. In other examples, the adaptor can be insert molded or co-molded with the needle guard and the adaptor body can incorporate surface features, such as tabs, voids, and/or barbs, to enable molding the proximal wall with the adaptor body and providing surfaces for the plastic to bond or grip with.

In embodiments, the side surface of the adaptor and the side surface of the proximal wall can be adjacent one another. That is, the side surface of the adaptor and the side surface of the proximal wall form a generally even outer surface profile. In embodiments where the adaptor body and the proximal wall can be understood as generally cylindrical, the side surface of the adaptor and the side surface of the proximal wall can be understood as having substantially the same diameter, which can define the outer diameter or outer dimension of the combined proximal wall of the needle guard and adaptor. The locating protrusions on the engagement tabs can define an outer-most diameter of the combination needle guard and adaptor that is larger than the outer dimension of the combined proximal wall.

In an example, the engagement tabs of the adaptor extend in the proximal direction. The adaptor, with the engagement tabs, can attach to the proximal wall of the needle guard and the engagement tabs extend in the proximal direction to form a hybrid clip having at least two different materials. It can be understood that the adaptor could be oriented in the opposite direction such that the engagement tabs extend in the distal direction of the combined proximal wall. In the case of the engagement tabs extending in the distal direction, the engagement tabs can extend distally over the side surface of the proximal wall of the needle guard.

The adaptor can be formed from metal. In some embodiments, the adaptor can be formed by stamping and bending a metal sheet to form the engagement tabs and the locating detents. In some embodiments where the proximal wall of the needle guard is made of plastic, the proximal wall can be co-molded or insert molded with the adaptor. Furthermore, in such cases, the stamping and bending of the metal sheet can provide for bonding tabs extending distally from the distal surface. These bonding tabs can facilitate bonding with the plastic of the proximal wall by either providing a contact surface for insert molding or to provide a feature for snap fitting to the proximal wall.

Alternatively, in some examples but not claimed the proximal wall of the needle guard can be formed with engagement tabs. That is, the proximal wall could serve as the adaptor body, making it a unitary construction. As such, not claimed examples of this type would not require a separate adaptor. Instead, the proximal wall of the needle guard itself may have the engagement tabs extending from its side surface. For example, the proximal wall of the needle guard can be insert molded with a plurality of metallic engagement tabs, and wherein the engagement tabs are not connected to or is not part of any separate metallic body.

A hybrid clip described herein is understood to mean a spring clip, a needle guard, or a needle protective device that is made from at least two different materials that can be used with a needle with a sharpened tip and is configured to block the needle tip to prevent inadvertent needle sticks. The hybrid clip can be located inside a catheter hub with the locating protrusions of the engagement tabs of the adaptor contacting the interior surface of the catheter hub to retain the needle guard inside the catheter hub in the ready to use position.

The needle can have a needle tip extending out a distal opening of the catheter tube and the needle can attach to the needle hub at the proximal end of the needle. In some examples, the hybrid clip is located in a third hub, which is distinct from the catheter hub and the needle hub. An exemplary IV catheter assembly with a third hub having a needle guard located therein is disclosed in U.S. Pat. No., 8,591,468.

In the ready position, a needle cannula is inserted through the opening of the adaptor, the opening of the needle guard, the catheter hub, and the catheter tube. Due to the positioning of the needle through the openings of the adaptor and the needle guard in the ready to use position, the two arms of the needle guard, or one arm if only one is incorporated, are deflected radially outward by the presence of the needle. Due to this deflection, the two arms can have a restoring force acting on the needle. In other examples, the arms can be provided or reinforced with metal strips or arms to increase the restoring forces. Optionally, the arms can be provided with an elastic band and the elastic band provides the added restoring force.

Upon withdrawal of the needle after successful venipuncture, when the needle is moved proximally of the distal walls, the resilient arms are no longer biased by the needle and can move radially inwardly towards the axis X of the needle guard. This then allows the distal walls to cover the needle tip.

Due to a change in profile of the needle, such as a crimp, weld, sleeve, or material build-up, further withdrawal of the needle in the proximal direction would result in the change in profile of the needle engaging the perimeter defining the opening on the proximal wall or the opening of the adapter. Preferably the engaging perimeter for interacting with the change in profile is metal, in order to increase the force to pull the change in profile through the opening and the needle guard distally off of the needle tip.

In an example, both openings on the proximal wall and the adaptor are smaller than the largest dimension of the change in profile. In another example, the opening on the proximal wall can be larger than the largest dimension of the change in profile but the opening on the adaptor is smaller than the largest dimension of the change in profile. At this point, further withdraw of the needle in the proximal direction will also withdraw the needle guard in the proximal direction along with the needle. As the needle tip moves proximally of the two distal walls, the needle tip will be covered and protected upon withdrawal from the catheter hub. In other examples, other hybrid clips described elsewhere herein can be used with a catheter hub assembly.

A variation of the needle guard and adaptor can include having the engagement tabs folded to point in the distal direction. That is, the tabs have free ends that point in the distal direction compared to other embodiments in which the free ends point in the proximal direction.

The adaptor can be configured to attach at a proximal end of the needle guard. The engagement tabs can be bent so that the free ends extend or point in the proximal direction. In some embodiments, the engagement tabs have free ends that point in the distal direction. When secured to the proximal wall of the needle guard, the engagement tabs extend distally over the side of the proximal wall even though the adaptor is located proximally of the proximal wall. As such, the contact points of the locating protrusions can be located distal of the adaptor body when located inside an interior of a catheter hub. The contact points of the locating protrusions can also be located distally of the proximal wall of the needle guard when located inside an interior of a catheter hub.

In some embodiments, the adaptor only has two engagement tabs extending from the side surface of the adaptor. The two engagement tabs can be equally spaced from one another around the side surface. In other examples, there are three engagement tabs equally spaced apart or unequally spaced apart around the perimeter of the proximal wall.

In an embodiment, the adaptor can be made from a metal material for fitment to the proximal wall of the needle guard from a position distal of the proximal wall. The adaptor can be understood as a variant allowing for fitment to the proximal wall from a position distal of the proximal wall. The adaptor body can have two opposed cut outs on the adaptor body. The adaptor body can have a shape that is other than round or circular. Each cut out can extend from the side surface inward to an intermediary position between the side surface. A central cross member can separate the two cut outs. The two opposed cut outs can provide openings for the resilient arms of the needle guard to pass around the adaptor body when the adaptor is positioned distally of the proximal wall. That is, the adaptor can be attached such that the proximally facing surface of the adaptor contacts the distally facing surface of the proximal wall of the needle guard.

With the opposed cut outs of the adaptor body, the adaptor body has a shape that resembles the letter "H". Said differently, the adaptor body can have two generally upstanding branches that are connected to one another by a generally horizontal branch. The adaptor body can have a central cross member defined by the opposed cut outs. A perimeter defining an opening can be located at the central cross member of the adaptor body. Each end of the central cross member can be attached to an upstanding structure or frame portion defining the side surface of the adaptor body. As such, the central cross member, the frame portions, and the cut outs can collectively define the cylindrical shape of the adaptor body.

The engagement tabs can extend from the frame portions and extend in the proximal direction. The engagement tabs can be equally spaced around the circumference defined by the side surface. There can be one or more engagement tabs. In an example, there can be four engagement tabs. However, the embodiment can be practiced with just two engagement tabs, as further discussed below.

In an example, the adaptor is attached to the needle guard and the adaptor body defined by the central cross member and the frame portions are in contact with a distal surface of the proximal wall. The engagement tabs can extend in the proximal direction such that the locating protrusions are proximal of the adaptor body of the adaptor. In some embodiments, the locating protrusions can be located at least partially over the proximal wall of the needle guard.

In an example, the adaptor body of the adaptor is located distal of the proximal wall of the needle guard and the engagement tabs extend in the distal direction. The engagement tabs can be located distally of both the proximal wall of the needle guard and the adaptor body.

An adaptor can attach to the distal surface of the proximal wall of the needle guard. However, the engagement tabs can be bent to extend in the proximal direction. In an example, the adaptor is attached on the distal surface of the proximal wall but wherein the bends are opposite, allowing the engagement tabs to extend in the distal direction. The engagement tabs can extend distally or in the distal direction such that the contact point of the locating protrusions located on the engagement tabs can be located distal of both the adaptor body and the proximal wall of the needle guard.

In an example, the adaptor, which can be made from a metal material, only has two engagement tabs extending from the adaptor body of the adaptor, such as from the side surface of the adaptor body. The two engagement tabs can be equally spaced from one another around the side surface. In other embodiments, the engagement tabs can be offset from the positioning of the arms by 90 degrees. That is, the two resilient arms and the two engagement tabs are preferably equally spaced apart from one another. However, the spacing can be un-even or random, although less preferred.

In an example, the adaptor is located distal of the proximal wall of the needle guard but spaced apart from the proximal wall. This design does not require co-molding or bonding of the metallic adaptor against the plastic proximal wall but the two can be co-molded or insert molded. The adaptor can be positioned at an intermediary position along the arms between the proximal wall and the one or more distal walls. The adaptor can contact an interior of a catheter hub with its locating protrusions locating on respective engagement tabs to maintain its position. The placement of the central cross member of the adaptor can also aid in fixing the position of the needle guard and increasing the force required to pull the needle guard distally off the needle tip.

In operation with the hybrid guard mounted onto a needle, withdrawal of the needle proximally relative to the hybrid guard can force the adaptor to move in the proximal direction due to the interaction between a change in profile of the needle engaging the perimeter defining the opening of the adaptor as the needle retracts in the proximal direction relative to the hybrid guard. Accordingly, upon retraction of the needle, the adaptor can move in the proximal direction with the needle to move against the proximal wall for maintaining the coupling of the needle guard to the adaptor. In some embodiments, the diameter of the opening of the adaptor can be smaller than the opening of the proximal wall on the needle guard.

In an example, the adaptor can be made from a metal material and attached to a needle guard so that the biasing plate is located radially outward relative to a respective resilient arm of the needle guard. The adaptor can include an adaptor body, engagement tabs extending from the adaptor body, such as from a side surface of the adaptor body, and a biasing plate extending from the adaptor body.

A locating protrusion can be located on each engagement tab. In some embodiments, the adaptor body can have a partial circular shape corresponding to a portion of the proximal wall of the needle guide, between one of the resilient arms and the side surface of the proximal wall. In an example, the engagement tabs can extend from the side surface of the adaptor body of the adaptor in a first direction and the biasing plate can extend from the side surface of the adaptor body of the adaptor in a second direction, opposite the first direction. In other examples, the engagement tabs and the biasing plate can extend from the adaptor body in the same direction. In an example, the engagement tabs can extend from the arc side edge of the adaptor body while the biasing plate can extend from a bottom chord of the partial circle of the adaptor body.

The biasing plate of the adaptor can be configured to bias a resilient arm of the needle guard towards the needle shaft, when in used with a needle. The adaptor, with the biasing plate, can be configured to hold the needle guard inside the catheter hub, with the aid of the engagement tabs. In an example, two adaptors of the present embodiment can be used with a needle guard. When the resilient arms of the needle guard are biased radially outward by the presence of a needle in a ready to a use position, the biasing plate on each adaptor can also be biased outwardly and has a restoring force. As such, the bias provided by the two biasing plates on the two resilient arms of the needle guard ensure that the resilient arms will move to a blocking position and cover the needle tip even if the arms are plastic and have taken a set during storage.

In an, the hybrid clip comprises a needle guard, which in an embodiment can have two resilient arms and two adaptors, each adaptor comprising an adaptor body, a biasing plate, and at least one engagement tab, which can extend in a distal direction or a proximal direction from the adaptor body.

In some embodiments, the partial circle of the adaptor body, such as the proximally facing surface of the adaptor body, can be used for bonding or affixing with the distally facing surface of the proximal wall of the needle guard. Optionally, one or both adaptors can be co-molded or insert molded with the needle guard.

In an example but not claimed, two adaptors, one for each of the two resilient arms, is incorporated with a needle guard. As shown, each adaptor has an adaptor body that attaches to the proximal wall and the biasing plate extends over the outer surface of the respective arm. The engagement tabs extend over the side of the proximal wall in the proximal direction.

A center adaptor plate can be incorporated to reinforce the opening on the proximal wall of the plastic needle guard. In an example, the center adaptor plate, which can be made from a metal material, can be generally rectangular in shape and comprises a perimeter defining an opening. The opening on the center adaptor plate can be smaller than the largest dimension of a change in profile so that when used with a needle, proximal movement of the needle relative to the hybrid clip will cause the change in profile to engage the opening. With further proximal movement and after the engagement, the change in profile can cause the hybrid clip to then move in the proximal direction with the needle. The center adaptor plate can attach to the proximal wall via adhesive or bonding, or by insert or co-molding.

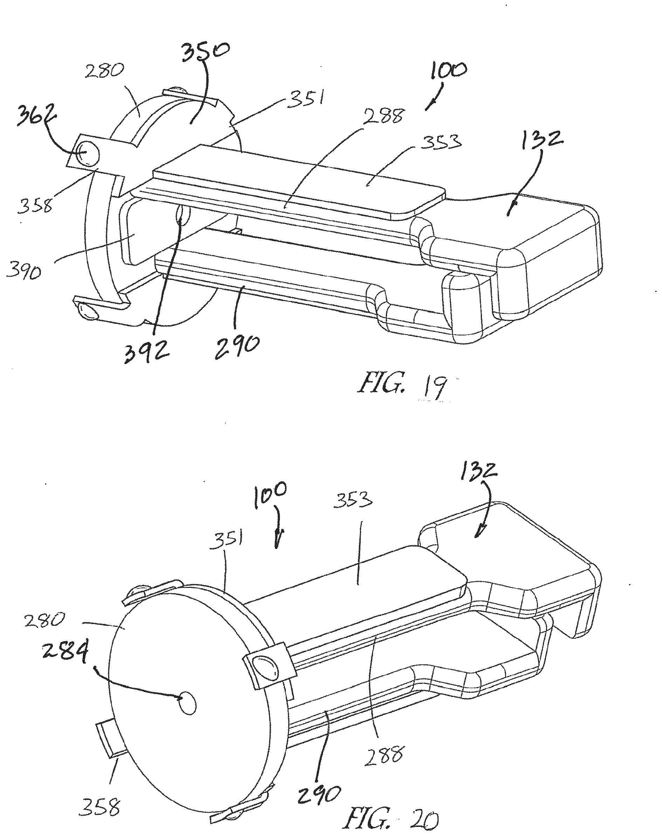

In an alternative example but not claimed, three metal inserts of FIGs. 19 and 20, the two adaptors, and the center adaptor plate can be formed as a one-piece structure and assembled or bonded to the needle guard in a similar manner as described elsewhere herein. Preferably the one-piece adaptor or the multi-piece adapters are only bonded or welded or insert molded to the proximal wall and not to the resilient arms. This allows the biasing plate to flex independently of the resilient arm.

In an example but not claimed, a hybrid clip having a needle guard and two adaptors is provided wherein each adaptor is located distal of the proximal wall of the needle guard and the adaptor body is spaced apart from the proximal wall. The biasing plate can be fixed to the respective resilient arm of the needle guard by means of adhesive.

The engagement tabs may optionally be bent so that the free ends of each engagement tab extend in the distal direction or in the proximal direction.

In some embodiments, a hybrid clip of the present invention can have at least one adaptor with at least two engagement tabs, and wherein at least one of the engagement tabs extends in the proximal direction and at least one of the engagement tabs extends in the distal direction. A hybrid clip not forming part of the present invention can have two adaptors with each adaptor having at least one engagement tab, and wherein at least one of the engagement tabs extends in the proximal direction and at least one engagement tab extends in the distal direction.

A needle guard can be formed from a single piece of material. Optionally, one or more wall guards made from a different material than the needle guard may be incorporated. The wall guards can be configured to be placed on a wall or on wall sections of the needle guard. In instances where the wall guards are placed on the distal walls of the needle guard, the wall guards may be referred to as distal wall guards.

In some embodiments, the metal material used for can be stainless steel or other medical grade metallic materials in order to have good elastic properties. However, alternative materials can be considered, including plastics, such as engineered plastics like PEEK and PEK, and composites. The basic shape of a needle guard stamped for bending into a needle guard can include a center portion, which can serve as a proximal wall having an opening. In an embodiment, the center portion that forms the proximal wall has a generally rectangular shape with other shapes contemplated.

On two opposed sides of the center portion can extend strips of material corresponding to the resilient arms. At end portions of the resilient arms, at least one pointed tooth, or two pointed teeth, or other retention features, can be stamped or otherwise formed into the material. On the other opposed sides of the center portion, there can extend strips of material corresponding to engagement tabs. Locating projections can be formed on each strip by coining or pressing a structural feature against an anvil.

Each of the strips on the stamped material sheet can be bent in at least two locations to form the resilient arms and the engagement tabs. A first bend can be performed near the center portion for each longer strip. For the resilient arms, a second bend can be performed at an intermediary location along the strip of material to form a distal wall. In this way, the resilient arms can be formed with distal walls pointing towards one another.

In an example, the needle guard with the center portion can have first and second arms that can be longer than a pair of engagement tabs.

The distal wall guards may be made from a soft plastic having a low coefficient of friction, such as low-density polyethylene (LDPE) or high-density polyethylene (HDPE). In use with a needle located between the two distal wall guards, the low coefficient of friction reduces the force necessary to withdraw the needle in the proximal direction relative to the two distal walls to allow the distal walls to block the needle tip from inadvertent needle sticks. The use of such low coefficient of friction guards can help with fixation of the needle after withdrawal by lessening sliding of the needle tip on the needle guard compared to the needle tip contacting or hitting against a metallic surface. Instead, the needle tip can stick to the plastic wall guards when in the protected position instead of sliding laterally when hitting and pivoting against bare metal. The plastic caps can be extruded and cut to the width of the distal wall or molded, among other manufacturing methods. The distal wall guard preferably has a slit for fitment with a respective resilient arm, such as for receiving a distal wall of the resilient inside the space provided at the slit.

With the stamped teeth being angled away from the end of the resilient arm and also set at an angle from the rest of the resilient arm, the teeth can bite into the distal wall guard like barbs once the distal wall guard is put into place inside the slit. This arrangement of the teeth allows for a light assembly force while also providing sufficient holding force so that the distal wall guard does not come off during use. In some embodiments, the distal wall guard can be installed prior to bending of the stamped metal sheet to form the needle guard.

The resilient arms and the engagement tabs can extend in a distal direction from the proximal wall of the needle guard. In some embodiments, the engagement tabs can extend in the opposite proximal direction. However, it may be beneficial to have the engagement tabs extend in the distal direction such that they cover the needle from sight and also provide a physical barrier to restrict the needle tip from sliding laterally out of the resilient arms.

In terms of retention of the needle guard inside a catheter hub, the proximal wall of the needle guard can be used to engage with a catheter hub, such as potentially by wedging or engaging the engagement tabs with the interior of the catheter hub or against a Luer taper of the catheter hub.

A needle guard with two arms but only one distal wall for blocking the needle tip can be practiced. The needle guard can comprise a single strip of material stamped and bent to define a center portion or proximal wall and the two arms. The proximal wall can have a perimeter defining an opening for a needle to pass therethrough. Extending distally from the proximal wall are two resilient arms. The two resilient arms can be substantially parallel to one another. Alternatively, the two resilient arms can be set an angle relative to the other.

At the distal portion of the first resilient arm, a curved protrusion or hook can be incorporated. For example, the stamped metal sheet for forming the needle guard can be cold worked to form the curved protrusion or hook at an end of the resilient arm. The curved protrusion can include a first section and a second section. The first section can extend radially outwardly away from a lengthwise axis defined by the needle guard, and then terminated with a curved section to form the second section. The needle guard may have more than one curved protrusion of the same size or of different sizes.

The second resilient arm can incorporate a distal wall extending at an angle to the lengthwise section of the arm, downstream of a curved protrusion, which can have a first section and second section. In some embodiments, the distal wall can contact the curved protrusion of the first resilient arm. As such, when the needle guard of the present embodiment is in used and after a needle is withdrawn in a proximal direction relative to the needle guard following successful venipuncture, the single distal wall can move back to cover the needle tip.

The second arm can have a second curved protrusion at an end part of the distal wall. The second curved protrusion can be smaller in size than the curved protrusion on the first arm so that when the two arms move closer together in the used position to cover the needle tip from inadvertent needle sticks, the smaller second curved protrusion can recess within the larger profile of the larger curved protrusion on the first arm. In other example, the two curved protrusions have approximately the same size so that the distal wall is forced to deflect slightly outwardly in the distal direction when the needle guard is in the protected position.

In an example, the needle guard has two alternative distal wall guards. In the first distal wall guard embodiment, a plastic cap having a wall structure with a lengthwise, lateral, opening and a relief, which can have a three-sided cut-out. The cap can have two ends for receiving the two curved protrusions on the second arm, one upstream of the distal wall and one downstream of the distal wall. The lateral opening can slide laterally over the distal wall and the curved protrusions. The arm section that extends from the first section of the lower or upstream curved protrusion can be accommodated by or can project out through the relief formed with the distal wall guard.

The second distal wall guard embodiment can embody a plate. In a particular example, the second distal wall guard embodiment can resemble a wedge or a wedge shape structure along a side cross-section. The distal wall guard can be a solid piece of plastic to be inserted on the proximal side of the distal wall and held in place by fitment with the upstream and downstream curved protrusions at the distal wall. In some embodiments, the plastic insert for the distal wall guard can be taken from a roll, inserted into the clip from the side and then cut off from the rest of the roll. The distal wall guards may be held in place with contact with teeth.

In an example, the insert can be made from a plastic material. The plastic insert may be a tube secured to, attached to, or bonded to the proximal wall and extending distally from the proximal wall, between the two arms. The insert can have a lumen or bore and the bore can be in communication or aligned with the through opening of the proximal wall. In some embodiments, the insert may be a cylinder with other outer surface geometries contemplated.

In some embodiments, the insert may have clips and for clipping attachment to the needle guard. In an example, a stamped plate can be formed with the insert, such as co-molded, insert molded, or bonded with the insert. The stamped plate can then fold around the proximal wall to secure insert to the proximal wall. The stamped plate having the clips can include a perimeter defining an opening for passing the needle therethrough. The opening on the stamped plate can have a smaller dimension than the largest dimension of the change in profile on the needle to enable the change in profile to engage the opening. The insert can be configured to guide the needle guard and the needle during relative movement to limit or strict side-to-side or pivoting movement of the needle relative to the needle guard.

In some embodiments, the insert can have a bore with a bore diameter that is slightly larger than the maximum diameter of the change in profile of the needle so that the crimp can be retracted to the metal proximal wall of the stamped plate to engage the opening. Alternatively, the bore of the plastic insert can also be smaller than the maximum diameter but slightly larger than a diameter of the needle shaft. In some embodiments of the latter case, the plastic insert can be placed on the proximal side of the proximal wall and it can be shorter as the clearance between the needle shaft and the guide bore can be kept to a minimum.

Methods of making and of using needle guards, adaptors for use with needle guards, needle assemblies and catheter assemblies for use with needle guards, including hybrid clips, and components thereof as described herein are within the scope of the present disclosure. The methods are understood to include the use of a catheter assembly and withdrawing the needle such that surfaces of the needle guard are located to a side of the needle and then movable distal of the needle tip to cover the tip of the needle from inadvertent needle sticks.

### BRIEF DESCRIPTION OF THE DRAWINGS

These and other features and advantages of the present devices, systems, and methods will become appreciated as the same becomes better understood with reference to the specification, claims and appended drawings wherein:
FIG. 1 shows a perspective view of a needle guard from a distal end.
FIG. 2 shows a perspective view of the needle guard of FIG. 1 from the proximal end.
FIG. 3 shows an exploded perspective view of the needle guard from the distal end with an adapter.
FIG. 4 shows an exploded perspective view of the needle guard of Fig. 3 from the proximal end.
FIG. 5 shows a perspective view from the distal end of the needle guard of Figs. 3 and 4 attached to the adaptor.
FIG. 6 shows a perspective view of the needle guard and the adaptor of Fig. 5 from the proximal end.
FIG. 7 shows a cross-sectional side view of a hybrid clip located inside a catheter hub 106 with the locating protrusions of the engagement tabs of the adaptor contacting the interior surface of the catheter hub to retain the position of the needle guard in the ready to use position.
FIG. 8 shows an exploded perspective view from the distal end of another embodiment.
FIG. 9 shows an exploded perspective view from the proximal end of the embodiment of Fig. 8.
FIG. 10 shows a perspective view from the distal end of the needle guard attached to an alternative adaptor.
FIG. 11 shows a perspective view of the needle guard and the adaptor of Fig. 10 from the proximal end.
FIG. 12 shows a perspective view of the alternative adaptor of Figs. 10 and 11 from the proximal end.
FIG. 13 shows a perspective view of an alternative needle guard from the distal end.
FIG. 14 shows a perspective of the needle guard of Fig. 13 view from the proximal end.
FIG. 15 shows an embodiment of the needle guard and the adaptor of FIGs. 10 - 12 where the adaptor is distally spaced apart from the proximal wall of the needle guard from the distal end.
FIG. 16 shows the embodiment of FIG. 15 from the proximal end.
FIG. 17 shows a perspective view of an adapter having a biasing plate from the proximal end.
FIG. 18 shows a top plan view of a metal stamping that can be bent to form the adaptor of Fig. 17.
FIG. 19 shows a perspective view from the distal end of the adapters of Fig. 17 and a central plate attached to the needle guard from the distal end.
FIG. 20 shows a perspective view of the needle guard and the adaptor of Fig. 19 from the proximal end.
FIGs. 21 and 22 show exploded views of the needle guard and adaptors of FIGs. 19 and 20 without the center adaptor plate
FIGs. 23A-23C show a needle guard formable from a single piece of material with the exception of a distal wall guards made of a second material.
FIG. 24 shows distal wall guards from a top and bottom perspective.
FIG. 25 shows a cross-sectional side view of an embodiment of a needle guard with two arms but only one distal wall for blocking the needle tip.
FIG. 26 shows a perspective view from the proximal side of the needle guard of FIG. 25.
FIG. 27 shows an exploded perspective view of the needle guard of FIGs. 25 and 26 with two alternative plastic caps for placement either over the single distal wall or on the inside surface between the bottom elbow and the upper hook.
FIG. 28 shows a cross-sectional view of an alternative needle guard.
FIG. 29 shows a perspective view of the needle guard of Fig. 29 from the proximal end.

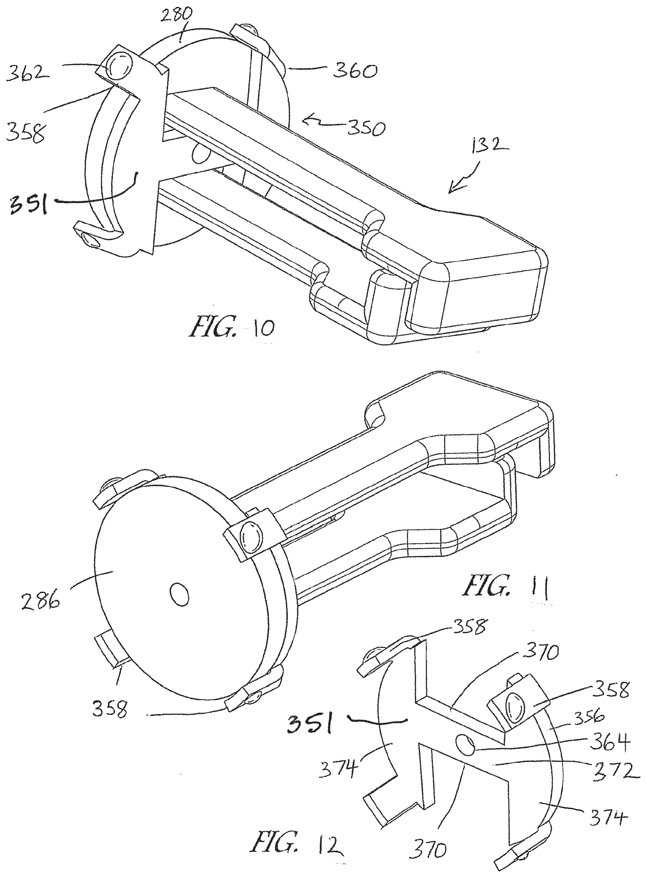

Figures 10-16 depict needle guards according to the present invention.

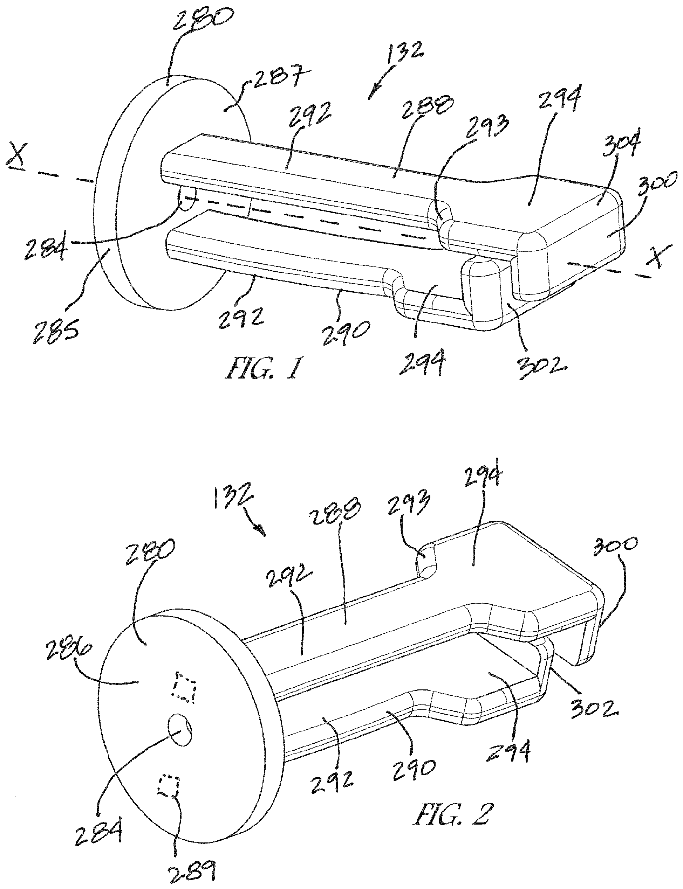

Figures 1, 2 and 23A-29 depict elements of the needle guard that may be used in the present invention.

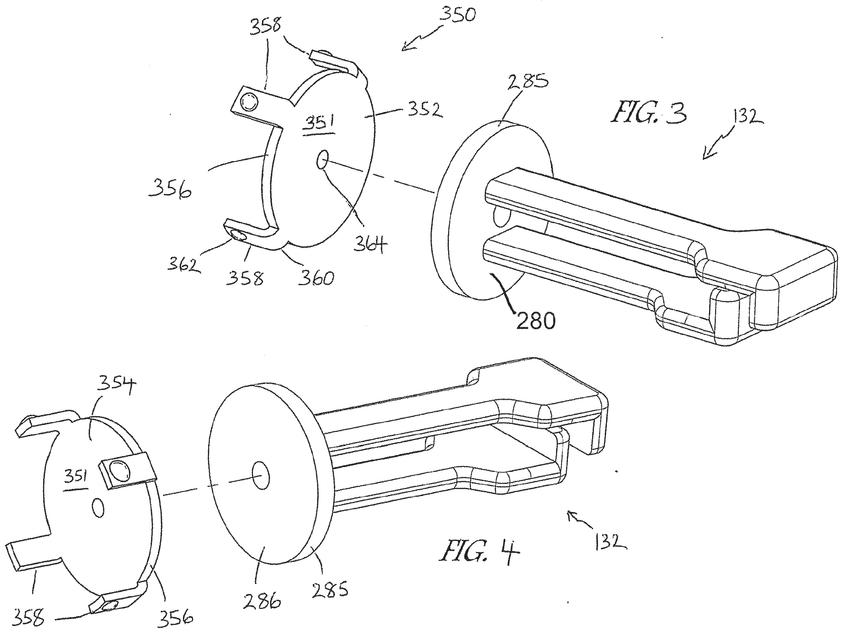

Figures 3-9 and 17-22 depict exemplary needle guards not forming part of the present invention.

### DETAILED DESCRIPTION

**As** denoted elsewhere herein, like element numbers are intended to indicate like or similar elements or features.

FIG. 1 illustrates a perspective view of a needle guard 132 from a distal end. FIG. 2 illustrates a perspective view of the needle guard 132 of FIG. 1 from a proximal end. The needle guard 132 may be sized and shaped to fit in an interior of a catheter hub have standard hub dimensions, such as a Luer opening. Optionally, the needle guard 132, which may alternatively be referred to as a safety spring clip or a needle protective device, may be used with a needle or trocar without a catheter hub. The needle guard 132 has a proximal wall 280 and at least one needle guard arm, or arm for short. As shown in FIG. 1, in some embodiments, the proximal wall 280 may be cylindrical, or disc-like, in shape. The proximal wall 280 may be defined by a cylindrical side surface 285, a proximally facing wall surface 286, and an opposed distally facing wall surface 287. The proximal wall 280 may be sized and shaped to correspond with an interior of a catheter hub. Alternative shapes of the proximal wall 280 may provide for a different cross-sectional shape from the interior of the catheter hub while fitting within the interior of the catheter hub, or the proximal wall 280 may have a cross sectional shape corresponding to an interior of the catheter hub. As used herein and within the medical device industry, the term distal end connotes the end further away from the user or practitioner and the proximal end is opposite the distal end or closer to the user.

The proximal wall 280 has an opening 284 defined by a perimeter. The opening 284 can be sized and shaped to allow a needle or cannula to pass through the proximal wall 280. If the needle incorporates a crimp, a sleeve, or a material buildup, which can generally be referred to as a change in profile, the diameter of the opening 284 can be smaller than the change in profile so that there is an interference between the opening, or the perimeter of the opening, and the change in profile.

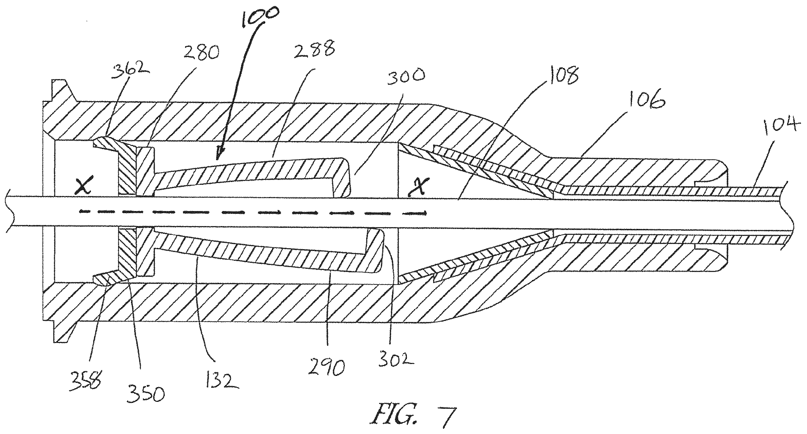

As understood in embodiments of IV catheter assemblies, such as shown in PCT/EP2016/069619, published as WO/2017/029361, the needle, which is attached to a needle hub, can be inserted through the opening 284 of the proximal wall 280 such that it can protrude from a distal opening of a catheter tube when the needle guard 132 is assembled in an IV catheter assembly in a ready to use position. An exemplary embodiment and description of a catheter assembly is shown below with respect to FIG. 7, and thoroughly described in the incorporated reference. The needle can have a change in profile, (not shown in Fig. 7) which can be distal of the catheter hub 106 in the ready to use position. The change in profile can be a crimp or a bulge that has a greater width or diameter than a diameter of the opening 284 as previously described. As such, when the needle is withdrawn or retracted in a proximal direction relative to the needle guard 132 following successful venipuncture, the change in profile catches and engages the perimeter of the opening 284 of the proximal wall 280. In doing so, the change in profile on the needle prevents the needle from separating from the needle guard 132 when the needle is retracted in the proximal direction, or prevents the needle guard from displacing distally off of the needle.

As shown in FIG. 1, two guard arms 288, 290 extend distally of the proximal wall 280. The two guard arms 288, 290 can be referred to as a first arm 288 and a second arm 290. The terms "first arm" and "second arm" serve to distinguish the two arms by name but not structurally distinguishing unless the context indicates otherwise. The two arms 288, 290 can each include sections of different arm widths. The first arm 288 and the second arm 290 can each include a first arm section 292 of a first width and a second arm section 294 of a second width, which is greater than the first width. A first end of the first arm section 292 can extend from the proximal wall 280 and a second end of the first arm section 292 can transition to the second arm section 294. The two arms 288, 290 can each have a first substantially uniform thickness. In embodiments, both of the two arms 288, 290 can have a substantially uniform width instead of the first width and the second width.

In an embodiment, the needle guard 132 can be formed from a plastic material. For example, the needle guard can be formed by plastic injection molding. The two arms 288, 290 can be molded so that they are hinged from the proximal wall 280 and are normally curved or pointed in the direction of the lengthwise axis X so that when the arms are spread outwardly away from one another, such as when the needle guard is mounted onto a needle, the two arms bias inwardly towards one another, such as having a restoring force to move towards one another. In other examples, metal inserts can be co-molded or insert molded with the two arms to increase the restoring force of the two arms. In still other examples, adapters or attachments can be incorporated with the two arms to increase their restoring force, as further discussed below.

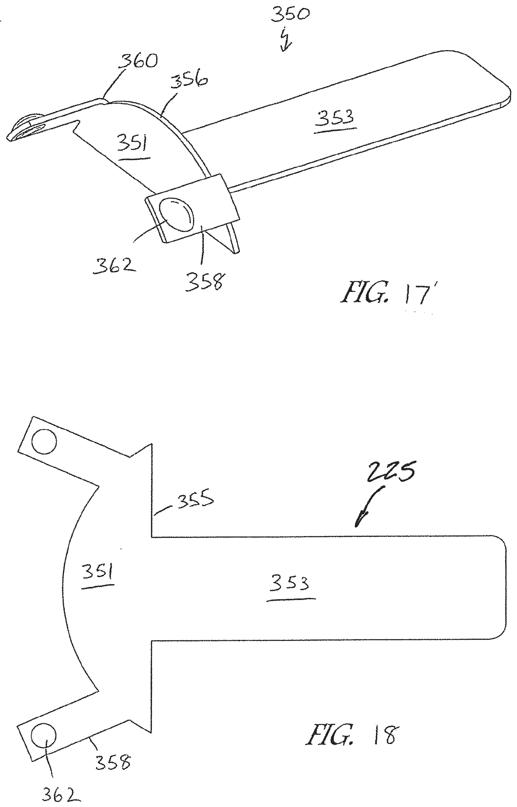

In some embodiments, the first arm section 292 and the second arm section 294 can have a stadium shape cross sectional shape, which can be understood as a geometric shape constructed of a rectangle with semicircles at a pair of opposite corners. However, the cross-sectional shape can embody other geometries, such as rectangular, oval, elliptical, polygonal, irregular, etc. In some embodiments, the first arm section 292 and the second arm section 294 can have a substantially uniform thickness and the first width of the first arm section 292 can transition to the second width of the second arm section 294 with an increasing width tapering section 293. As such, in embodiments, each of the two arms 288, 290, which can be resilient, can be understood as having a narrow first arm section 292 extending distally from the proximal wall 280. The arms 288, 290 can then increase in width in the tapering section 293 as they extend distally from the proximal wall 280. The second arm section 294 can thereby be a distal portion of the arms 288, 290 that is wider than the first arm section 292. As used herein, the arms being resilient is understood to mean being able to spring back into shape. For example, when the two arms are spread apart by the needle, the resilient arms can spring back after retraction of the needle proximally of the one or more distal walls of the needle guard so that the arms can block the needle tip. As further discussed below, such as with reference to FIGs. 17-22, the resiliency of the first am 288 and the second arm 290 can increase by incorporating an adaptor to each of the two arms. In some examples, only one of the two arms 288, 290 is equipped an adaptor.

The two arms 288, 290 can be circumferentially spaced around the opening 284 on the proximal wall. In some embodiments, the two arms 288, 290, which can be resilient, can be spaced 180 degrees from each other circumferentially around the opening 284. In other embodiments, the two arms 288, 290 can be spaced apart at different positions circumferentially from 180 degrees.

Two distal walls 300, 302 can be provided with the needle guard 132, one at an end of each of the two resilient arms 288, 290. Each of the distal walls 300, 302 can extend at an angle from the respective second arm section 294 of the two resilient arms 288, 290. The distal walls 300, 302 may extend towards each other. The two distal walls can have surfaces with lengths and widths that are sufficient to cover or block a needle tip. The surfaces can be located at or on two different side sections of the needle in a ready to use position and movable distal of the needle in a protective position to block the needle tip from inadvertent needle sticks. The lengths, which can be considered the direction extending from the respective second sections toward the axis X, can be selected so that the two distal walls overlap in a protective position. Alternatively, only one distal wall is incorporated, on one of the two arms, and the other arm can have a free end without a distal wall. Another alternative is a needle guard with only one arm with one distal wall at an end of the one arm.

The distal walls 300, 302 preferably overlap one another along the axis X of the needle guard 132 by utilizing different arm lengths of the two resilient arms 288, 290 and/or angling at least one of the distal walls 300, 302 at an elbow or diagonal section 304 between the distal wall and the resilient arm. As shown in FIG. 1, in embodiments, one arm can be longer than the other arm. In this way, the different lengths of the two arms 288, 290 allow for staggered, overlapping coverage along the axial direction X.

Alternatively, the two arms can be equal in length such that the distal walls 300, 302 abut one another or the distal walls can be angled from the second arm sections at different angles as they project toward the axis to allow for overlapping.

In fitment with an IV catheter hub of a catheter assembly, the arms 288, 290 are sufficiently flexible to allow for deflection radially outward such that the needle can extend through the opening 284 and extend distally between the two distal walls 300, 302 of the two arms 288, 290 when the IV catheter assembly is in the ready to use position. In the ready to use position, where the arms 288, 290 are deflected radially outward by the presence of the needle between the two distal walls, the arms 288, 290 preferably have a restoring force acting on the needle. Upon withdrawal of the needle after successful venipuncture, when the needle is moved proximal of the two distal walls 300, 302, the arms 288, 290, which can be resilient and have restoring forces, move back towards the axis X of the needle guard 132. In this way, the distal walls 300, 302 shield the needle from accidental finger pricks after withdrawal of the needle. The overlapping configuration of the two distal walls 300, 302 can restrict the needle tip from projecting between the two distal walls to re-emerge from the needle tip holding space proximal of the two distal walls.

In some embodiments, the two arms 288, 290 can each extend substantially parallel to one another. The two arms 288, 290 can each extend perpendicularly from the distally facing wall surface 287 of the proximal wall 280. The two arms can be singularly formed with the proximal wall and hinged off of the proximal wall.

In other embodiments, the two arms 288, 290 can each extend at an oblique angle relative to the distally facing wall surface 287 of the proximal wall 280. In embodiments, the two arms 288, 290 can each extend from the distally facing wall surface 287 at an angle ranging from 20 degrees to 89 degrees. More specifically, the two arms 288, 290 can each extend from the distally facing wall surface 287 at an angle ranging from 45 degrees to 80 degrees. The two arms 288, 290 may be angled such that they extend towards each other.

Although the embodiment shown in FIG. 1 shows that the two arms 288, 290 are adjacent to one another, other embodiments of the two resilient arms 288, 290 may have them intersect and cross over one another. For example, an embodiment where the two resilient arms intersect is shown in PCT/EP2016/069619.

In other embodiments, more than two guard arms may be used. For example, three or four or more guard arms may be incorporated with a needle guard of the present disclosure. The resilient arms may be equally spaced around the opening 284 at the distally facing wall surface of the proximal wall. Each of the guard arms can be arranged such that distal walls of the guard arms can overlap one another along the axial direction of the needle guard.

Embodiments of the needle guard 132 may have a proximal wall 280 and the guard arms 288, 290 made of different materials.

In some embodiments, the needle guard 132 may be made from plastic and metal materials. By using both plastic material and metal material, the needle guard 132 may be made in a more economical way by reducing the amount of metal needed.

For example, some embodiments of the proximal wall 280 may be made from a plastic material and the arms 288, 290 may be made from a metal material or be provided with both plastic and metal components, such as forming the arms as a hybrid. Using a metal material may can increase the biasing forces of the arms and increase shelf life as the metal will not set like some plastics might. In the case of some plastics, the plastic may set while being biased in the ready to use position due to a phenomenon called plastic creep. Creep is the tendency of a solid material to move slowly or deform permanently under the influence of mechanical stresses. Thus, including a metal component to the plastic component can maintain the resiliency of the overall hybrid structure even after prolonged storage or after periods of un-used.

Embodiments with the proximal wall 280 made from a plastic material and the arms 288, 290 made at least in part from a metal material may be formed by insert molding. For example, the proximal wall 280 may be insert molded around an end of the resilient arms 288, 280.

Alternatively, in some embodiments, the proximal wall 280 and the resilient arms 288, 290 may be manufactured separately. The proximal wall 280 may have slots 289 (FIG. 2) for the insertion of an end of the resilient arms 288, 290, which can be made from a metal material. In some embodiments, the ends of the resilient arms 288, 290 inserted into the slots 289 may extend past the proximal side of the proximal wall 280 from the distally facing wall surface and then bent, similar to a tab, in order to secure the resilient arms 288, 290 to the proximal wall 280. Optionally, adhesive or welding may be used to more securely attach the two arms 288, 290 to the proximal wall.

In some embodiments, one of the guard arms may be made from a metal material and another one of the guard arms may be made from a plastic material. In an example, the proximal wall 280 can be made out of metal and have slots 289, and the arms 288, 290 made from a plastic material and be inserted into the slots 289 in the proximal wall. In embodiments, the proximal end of the plastic arms 288, 290 could include at least one hook feature or projection in order to snap into the slots on the proximal wall and hook themselves fixedly to the proximal wall 280.

In other embodiments, the needle guard 132 may be formed from plastic materials by methods such as plastic injection. Embodiments of the needle guard formed from plastic material may have the proximal wall 280 and the arms 288, 290 unitarily formed. Alternatively, the proximal wall 280 and the arms 288, 290 may be separately formed and then attached together by plastic welding or adhesives. The arms can be provided with ribs or increased bulk mass extending at least partly along the length of each arm to increase the arm's restoring force.

FIGs. 3-7 illustrate an embodiment of a needle guard 132 attached to an adaptor 350. FIG. 3 illustrates an exploded perspective view of the needle guard and adaptor from the distal end. FIG. 4 illustrates an exploded perspective view of the needle guard and adaptor from the proximal end.

FIG. 3 shows an adaptor 350 (not claimed) for fitment with the needle guard 132. The adaptor 350 serves to fix the location of the needle guard 132 in the interior of the catheter hub as further described in FIG. 7 below. The adaptor 350 can have an adaptor body 351 having a distally facing surface 352, an opposed proximally facing surface 354, and a side surface 356. In some examples but not claimed, the adaptor body 351 can be generally sized and shaped to match the proximal wall 280 of the needle guard 132 but can be differently shaped. The adaptor 350 can be provided with an adaptor body and the proximal wall coupled to one another, as further discussed below. Additionally, the adaptor body 351 can have a perimeter defining an opening 364. The opening 364 on the adaptor can be aligned with the opening 284 of the proximal wall 280 of the needle guard, and the opening 364 of the adaptor 350 can also be sized and shaped to allow for the insertion of a needle but sufficiently small to interfere with a change in profile, such as a crimp or an enlarged portion, formed with the needle near the needle tip.

In some examples but not claimed, the adaptor body 351 can be generally cylindrical along an end view. The adaptor body 351 can correspond in shape and size to match the proximal wall 280. That is, the side surface 356 of the adaptor 350 can have a same diameter as the side surface 285 of the proximal wall 280 of the needle guard 132. In other embodiments, only parts or sections of the body 351 of the adaptor can have points that are coincident with the side surface 285 of the proximal wall. For example, a square adaptor body 352 can have four points that lie on the same outer surface profile as the side surface 285 of the proximal wall 280. At least two engagement tabs 358 can extend from adaptor body 351. In an example, the at least two engagement tabs 358 can extend from the side surface 356 of the adaptor 350. In the embodiment shown in FIGs. 3 and 4, there are four engagement tabs 358. However, there can be fewer than four but at least two or there can be more than four engagement tabs. The engagement tabs 358 are preferably equally spaced around the side surface 356 or can be randomly spaced around the side surface.

The engagement tabs 358 may join to the side surface 285 at an angle through a bend or elbow section 360. That is, the engagement tabs 358 can extend in the proximal direction due to the bend 360. In some embodiments, the bend 360 may be a 90-degree bend. As such, the engagement tabs 358 can be located at a position radially outward from the adaptor body 351. In some examples, the engagement tabs 358 are stamped from the same metal blank as the adaptor body 351 and then the bends or elbows 360 are simply formed as by-products in cold-working the tabs to point in the proximal direction.

Each of the engagement tabs 358 has at least one locating protrusion or projection 362. The locating projection 362 can project from a surface of the engagement tab 358. In an example, the locating projection 362 projects from the side or surface of the engagement tab that faces the interior of a hub housing, such as a catheter hub or a third hub. The at least one locating protrusion 362 can be oriented to contact a surface, such as the interior surface of a catheter hub. Thus, the engagement tab can face radially outward away from the axis X of the needle guard. The locating protrusion 362 may be a hemispherical protrusion or a portion of a sphere on the engagement tab. Alternatively, other geometric shapes could be used, such as a rectangular rib, a trapezoidal or half or partial oval shape. Preferably locating protrusion is smooth to avoid scratching the inside of the catheter hub. The locating protrusion 362 can be arranged to contact an interior of a catheter hub as described in FIG. 7 below. It can be envisioned that the locating protrusion 362 can be chosen in order to reduce the amount of surface contact and thereby reduce the frictional holding force of the engagement tabs 358 in a catheter hub. In other examples, the adaptor can be omitted and the locating protrusions 362 can be formed directly along the periphery of the side surface 285 of the proximal wall 280.

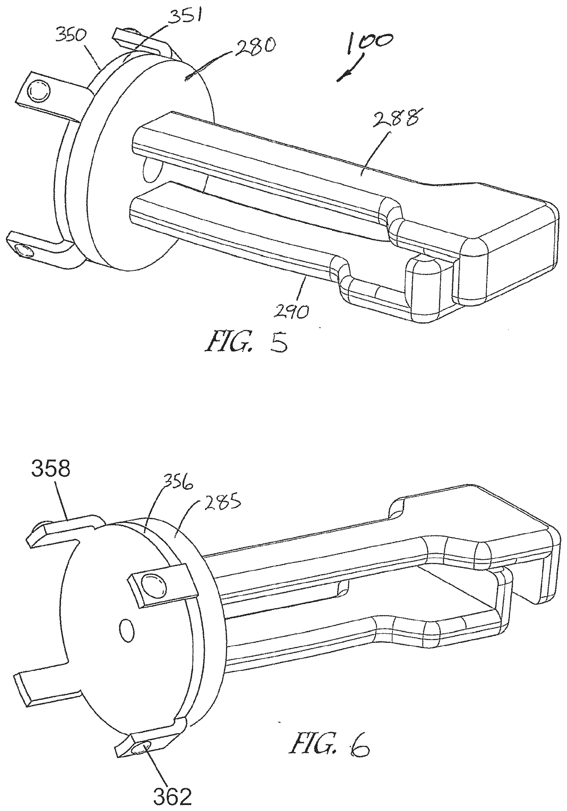

FIG. 5 illustrates a perspective view from the distal end of the needle guard 132 attached to the adaptor 350. The adaptor body 351 may be separately formed and then attached to the proximal wall, such as by welding or bonding. The distally facing surface 352 of the adaptor 350 can contact the proximally facing surface 286 of the proximal wall 280. In other examples, the adaptor 350 can be insert molded or co-molded with the needle guard and the adaptor body 351 can incorporate surface features, such as tabs, voids, and/or barbs, to enable molding the proximal wall 280 with the adaptor body 351 and providing surfaces for the plastic to bond or grip with.

FIG. 6 illustrates a perspective view of the needle guard 132 and the adaptor 350 from the proximal end. In examples but not claimed, the side surface 356 of the adaptor 350 and the side surface 285 of the proximal wall 280 can be adjacent one another. That is, the side surface 356 of the adaptor 350 and the side surface 285 of the proximal wall 280 form a generally even outer surface profile. In examples (but not claimed) such as that shown in FIG. 6 where the adaptor body 351 and the proximal wall 280 can be understood as generally cylindrical, the side surface 356 of the adaptor 350 and the side surface 285 of the proximal wall 280 can be understood as having substantially the same diameter, which can define the outer diameter or outer dimension of the combined proximal wall of the needle guard and adaptor. The locating protrusions 362 on the engagement tabs 358 can define an outer-most diameter of the combination needle guard and adaptor that is larger than the outer dimension of the combined proximal wall.

FIGs. 3-6 show the adaptor 350 with the engagement tabs 358 extending in the proximal direction. FIGs. 5 and 6 show the adaptor 350 with the engagement tabs 358 attached to the proximal wall 280 of the needle guard and the engagement tabs 358 extending in the proximal direction to form a hybrid clip 100 having at least two different materials. It can be understood that the adaptor 350 could be oriented in the opposite direction such that the engagement tabs 358 extend in the distal direction of the combined proximal wall. In the case of the engagement tabs 358 extending in the distal direction, the engagement tabs 358 can extend distally over the side surface 285 of the proximal wall 280 of the needle guard.

The adaptor 350 can be formed from metal. In some examples but not claimed, the adaptor 350 can be formed by stamping and bending a metal sheet to form the engagement tabs 358 and the locating detents 362. In some embodiments where the proximal wall 280 of the needle guard is made of plastic, the proximal wall 280 can be co-molded or insert molded with the adaptor 350. Furthermore, in such cases, the stamping and bending of the metal sheet can provide for bonding tabs extending distally from the distal surface 352. These bonding tabs can facilitate bonding with the plastic of the proximal wall 280 by either providing a contact surface for insert molding or to provide a feature for snap fitting to the proximal wall 280.

Alternatively, in some examples but not claimed, the proximal wall 280 can be formed with engagement tabs 358. That is, the proximal wall 280 could serve as the adaptor body 351, making it a unitary construction. As such, examples of this type would not require a separate adaptor 350. Instead, the proximal wall 280, such as that shown in FIG. 2, may have the engagement tabs 358 extending from its side surface 285. For example, the proximal wall 280 can be insert molded with a plurality of metallic engagement tabs 358, and wherein the engagement tabs are not connected to or is not part of any separate metallic body.

FIG. 7 is a cross-sectional side view of a hybrid clip 100, which is understood to mean a spring clip, a needle guard, or a needle protective device that is made from at least two different materials that can be used with a needle with a sharpened tip and is configured to block the needle tip to prevent inadvertent needle sticks. The hybrid clip 100 is shown located inside a catheter hub 106 with the locating protrusions 362 of the engagement tabs 358 of the adaptor 350 contacting the interior surface of the catheter hub 106 to retain the needle guard 132 inside the catheter hub 106 in the ready to use position. The hybrid clip 100 can be the clip shown in FIGs. 3-6. However, other hybrid clips described elsewhere herein can be used with the catheter assembly as shown and described. Although not shown, the needle 108 has a needle tip extending out a distal opening of the catheter tube 104 and is attached to the needle hub (not shown) at the proximal end of the needle. In some examples, the hybrid clip 100 is located in a third hub, which is distinct from the catheter hub and the needle hub. An exemplary IV catheter assembly with a third hub having a needle guard located therein is disclosed in U.S. Pat. No., 8,591, 468.

In the ready position, a needle cannula 108 is inserted through the opening of the adaptor 350, the opening of the needle guard 132, the catheter hub 106, and the catheter tube 104. Due to the positioning of the needle through the openings of the adaptor and the needle guard in the ready to use position, the two arms 288, 290 are deflected radially outward by the presence of the needle. Due to this deflection, the arms 288, 290 can have a restoring force acting on the needle. In other examples, the arms can be provided or reinforced with metal strips or arms to increase the restoring forces, or providing with an elastic band to add to the restoring force. Upon withdrawal of the needle after successful venipuncture, when the needle is moved proximally of the distal walls 300, 302, the resilient arms 288, 290 are no longer biased by the needle and can move radially inwardly towards the axis X of the needle guard 132. This then allows the distal walls 300, 302 to cover the needle tip.

Due to a change in profile of the needle, such as a crimp, weld, sleeve, or material build-up, further withdrawal of the needle in the proximal direction would result in the change in profile of the needle 108 engaging the perimeter defining the opening on the proximal wall 280 or the opening 364 (FIG. 3) of the adapter 350. Preferably the engaging perimeter is metal, in order to increase the force to pull the change in profile through the opening and the needle guard distally off of the needle tip. Thus, as in an example, both openings on the proximal wall 280 and the adaptor 350 are smaller than the largest dimension of the change in profile. In another example, the opening on the proximal wall 280 is larger than the largest dimension of the change in profile but the opening on the adaptor 350 is smaller than the largest dimension of the change in profile. At this point, further withdraw of the needle in the proximal direction will also withdraw the needle guard 132 in the proximal direction along with the needle 108. As the needle tip moves proximally of the two distal walls, the needle tip will be covered and protected upon withdrawal from the catheter hub 106. In other examples, other hybrid clips described elsewhere herein can be used with a catheter hub assembly as described with reference to FIG. 7.

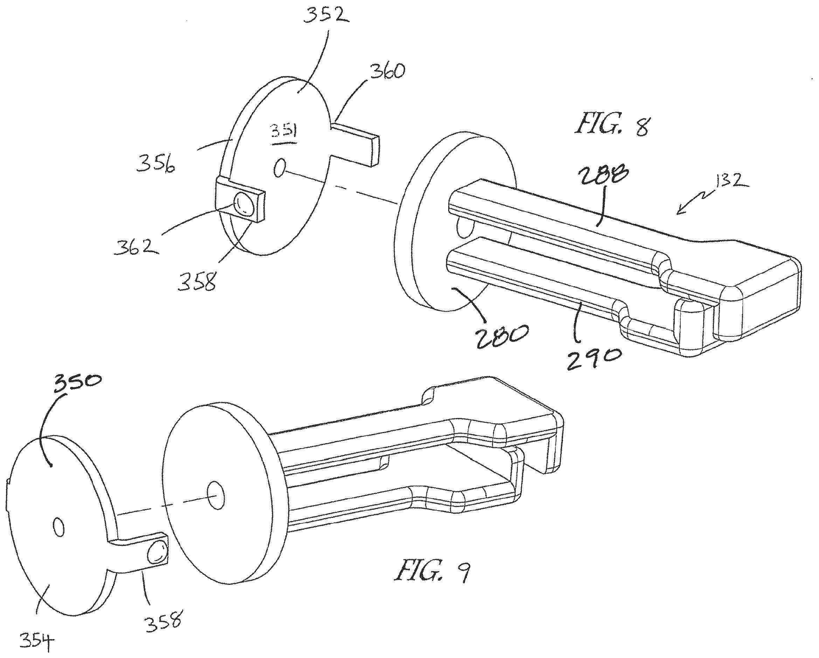

FIGs. 8 and 9 are exploded views of a variation of the needle guard 132 and adaptor 350 of FIGs. 3 and 4 with a variation on the engagement tabs 358 being folded to point in the distal direction. That is, the tabs have free ends that point in the distal direction compared to other embodiments in which the free ends point in the proximal direction. FIG. 8 illustrates an exploded perspective view of the needle guard and adaptor from the distal end. FIG. 9 illustrates an exploded perspective view of the needle guard and adaptor from the proximal end.

As shown in FIG. 8, the adaptor 350 is configured to attach at a proximal end of the needle guard 132. However, as explained above with respect to FIGs. 3-6, the engagement tabs 358 can be bent so that the free ends extend or point in the proximal direction. In the embodiment of FIG. 8, the bend 360 is opposite that of FIG. 3 where the engagement tabs 358 have free ends that extend in the proximal direction. Instead, the engagement tabs 358 have free ends that point in the distal direction. When secured to the proximal wall 280 of the needle guard 132, the engagement tabs 358 extend distally over the side of the proximal wall 280 even though the adaptor 350 is located proximally of the proximal wall 280. As such, the contact points of the locating protrusions 362 are located distal of the adaptor body 351 when located inside an interior of a catheter hub, such as that shown in FIG. 7. The contact points of the locating protrusions 362 can also be located distally of the proximal wall 280 of the needle guard when located inside an interior of a catheter hub.

In the embodiment of FIGs. 8 and 9, the adaptor 350 only has two engagement tabs 358 extending from the side surface 356 of the adaptor. The two engagement tabs 358 can be equally spaced from one another around the side surface 356.

FIGs. 10-12 illustrate an embodiment of the adaptor 350 according to the present invention, which can be made from a metal material, for fitment to the proximal wall 280 from a position distal of the proximal wall. FIG. 10 illustrates a perspective view from the distal end of the needle guard 132 attached to the adaptor 350. FIG. 11 illustrates a perspective view of the needle guard 132 and the adaptor 350 from the proximal end. FIG. 12 illustrates a perspective view of the adaptor 350 from the proximal end.

As shown in FIG. 12, the adaptor 350 is a variant allowing for fitment to the proximal wall 280 from a position distal of the proximal wall. The adaptor 350 is similar to the adaptor 350 of FIG. 3. However, the adaptor body 350 of the present embodiment has two opposed cut outs 370 on the adaptor body 351. Thus, the adaptor body 351 can clearly have a shape that is other than round or circular. Each cut out 370 extends from the side surface 356 inward to an intermediary position between the side surface 356. A central cross member 272 separates the two cut outs 370. The two opposed cut outs 370 providing openings for the resilient arms 288, 290 of the needle guard to pass around the adaptor body 351 when the adaptor 350 is positioned distally of the proximal wall 280. That is, the adaptor 350 can be attached such that the proximally facing surface of the adaptor 350 contacts the distally facing surface of the proximal wall 280 of the needle guard.

With the opposed cut outs 370 of the adaptor body 351, the adaptor body 351 has a shape that resembles the letter "H". Said differently, the adaptor body 351 can have two generally upstanding branches that are connected to one another by a generally horizontal branch. The adaptor body 351 has a central cross member 372 defined by the opposed cut outs 370. A perimeter defining an opening 364 can be located at the central cross member 372. Each end of the central cross member 372 can be attached to an upstanding structure or frame portion 374 defining the side surface 356 of the adaptor body 350. As such, the central cross member 372, the frame portions 374, and the cut outs 370 can collectively define the cylindrical shape of the adaptor body 351.

The engagement tabs 358 can extend from the frame portions 374 and extend in the proximal direction. The engagement tabs 358 can be equally spaced around the circumference defined by the side surface 356. There can be one or more engagement tabs 358. As shown in FIG. 12, there are four engagement tabs. However, the embodiment can be practiced with less, such as with just two engagement tabs, as further discussed below.

FIG. 10 illustrates an embodiment where the adaptor body of the adaptor 350 is distal of the proximal wall 280 of the needle guard 132. In this embodiment, the adaptor body 351 defined by the central cross member 372 and the frame portions 374 are in contact with a distal surface of the proximal wall 280. FIG. 11 illustrates that the engagement tabs 358 can extend in the proximal direction such that the locating protrusions 362 are proximal of the adaptor body 351 of the adaptor 350. In some embodiments, the locating protrusions 362 can be located at least partially over the proximal wall 280 of the needle guard 182.

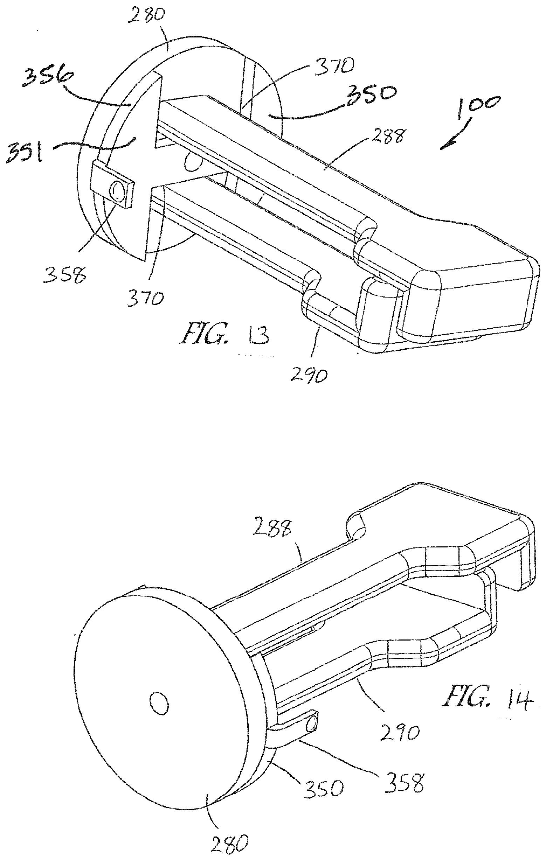

FIGs. 13 and 14 are perspective views of a variation of the needle guard 132 and adaptor 350 of FIGs. 10-12 with a variation on the engagement tabs 358 being folded in the distal direction. FIG. 13 illustrates a perspective view from the distal end. FIG. 14 illustrates a perspective view from the proximal end. As shown, the adaptor body 351 of the adaptor 350 is located distal of the proximal wall 280 of the needle guard and the engagement tabs 358 extend in the distal direction. The engagement tabs 358 are shown distal of both the proximal wall 280 and the adaptor body 351.

FIG. 10 therefore shows an adaptor 350 attached on the distal surface of the proximal wall 280 of the needle guard 132. However, as explained above with respect to FIGs. 3-6, the engagement tabs 358 of the FIG. 10 embodiment can be bent to extend in the proximal direction. In the embodiment of FIG. 13, the adaptor 350 is also attached on the distal surface of the proximal wall but wherein the bends are opposite to that of FIG. 10, allowing the engagement tabs 358 of the FIG. 13 embodiment to extend in the distal direction. The engagement tabs 358 extend distally or in the distal direction such that the contact point of the locating protrusions 362 located on the engagement tabs 358 are located distal of both the adaptor body 351 and the proximal wall 280.

In the embodiment of FIGs. 13 and 14, the adaptor 350, which can be made from a metal material, only has two engagement tabs 358 extending from the adaptor body 351 of the adaptor 350, such as from the side surface 356 of the adaptor body 351. The two engagement tabs 358 are equally spaced from one another around the side surface 356. In other embodiments, the engagement tabs 358 can be offset from the positioning of the arms 288, 290 by 90 degrees. That is, the two resilient arms 288, 290 and the two engagement tabs 358 are preferably equally spaced apart from one another. However, the spacing can be un-even or random, although less preferred.

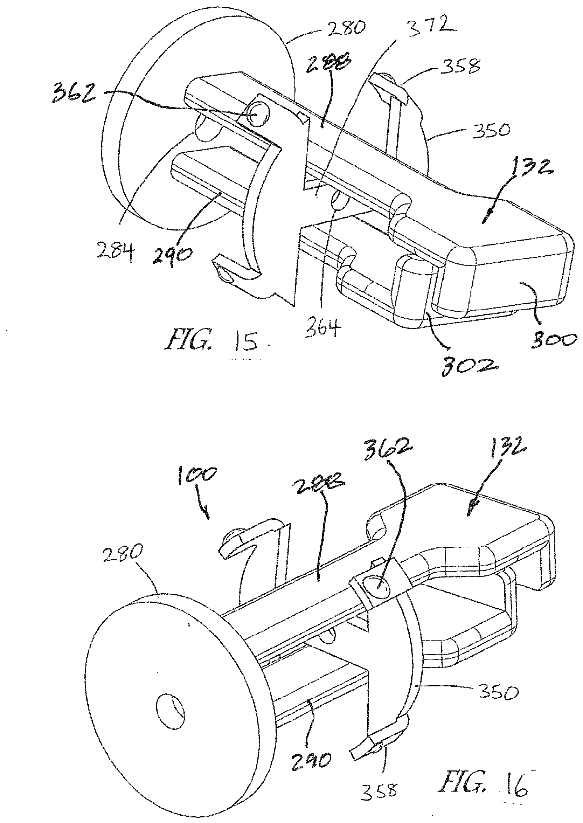

FIGs. 15 and 16 illustrate an embodiment of the needle guard 132 and the adaptor 350 where the adaptor 350 is distal of the proximal wall 280 of the needle guard and spaced apart from the proximal wall 280. This design does not require co-molding or bonding of the metallic adaptor against the plastic proximal wall but the two can be co-molded or insert molded. The adaptor 350 can be positioned at an intermediary position along the arms 288, 290 between the proximal wall 280 and the one or more distal walls 300, 302. The adaptor 350 can contact an interior of a catheter hub with its locating protrusions 362 locating on respective engagement tabs to maintain its position. The placement of the central cross member 372 of the adaptor 350 can also aid in fixing the position of the needle guard 132 and increasing the force required to pull the needle guard distally off the needle tip.

In operation with the hybrid guard 100 mounted onto a needle, withdrawal of the needle proximally relative to the hybrid guard 100 will force the adaptor 350 to move in the proximal direction due to the interaction between a change in profile of the needle engaging the perimeter defining the opening 364 of the adaptor 350 as the needle retracts in the proximal direction relative to the hybrid guard. Accordingly, upon retraction of the needle, the adaptor 350 will be moved in the proximal direction with the needle to move against the proximal wall 280 for maintaining the coupling of the needle guard 132 to the adaptor 350. In some embodiments, the diameter of the opening 364 of the adaptor 350 can be smaller than the opening 284 of the proximal wall 280 on the needle guard.

FIGs. 17-22 illustrate an example (not claimed) of an adaptor 350 having a biasing plate 353. FIG. 17 illustrates a perspective view of the adapter 350 of the present example (not claimed) from the proximal end. The adaptor 350 can be made from a metal material and attached to a needle guard so that the biasing plate 353 is located radially outward relative to a respective resilient arm 288, 290 of the needle guard. The adaptor 350 can include an adaptor body 351, engagement tabs 358 extending from the adaptor body 351, such as from a side surface 356 of the adaptor body, and a biasing plate 353 extending from the adaptor body 351. A locating protrusion 362 can be located on each engagement tab. In some embodiments, the adaptor body 351 can have a partial circular shape corresponding to a portion of the proximal wall 280 of the needle guard 132, between one of the resilient arms 288, 290 and the side surface 285 of the proximal wall 280. In an example, the engagement tabs 358 can extend from the side surface 356 of the adaptor body of the adaptor 350 in a first direction and the biasing plate 353 can extend from the side surface 356 of the adaptor body of the adaptor 350 in a second direction, opposite the first direction. In other examples, the engagement tabs 358 and the biasing plate 353 can extend from the adaptor body in the same direction. In an example, the engagement tabs 358 can extend from the arc side edge of the adaptor body while the biasing plate can extend from a bottom chord of the partial circle of the adaptor body 351.

FIG. 18 illustrates a top plan view of a stamped metal sheet 225 that can be bent to form the adaptor 350 of FIG. 17.

The biasing plate 353 of the adaptor 350 is configured to bias a resilient arm of the needle guard towards the needle shaft, when in used with a needle. The adaptor 350, with the biasing plate 353, is also configured to hold the needle guard inside the catheter hub, with the aid of the engagement tabs 358. In an example, two adaptors 350 of the present embodiment can be used with a needle guard. When the resilient arms of the needle guard are biased radially outward by the presence of a needle in a ready to a use position, the biasing plate 353 on each adaptor is also biased outwardly by and has a restoring force. As such, the bias provided by the two biasing plates 353 on the two resilient arms 288, 290 of the needle guard ensure that the resilient arms 288, 290 will move to a blocking position and cover the needle tip even if the arms are plastic and have taken a set during storage.

FIG. 19 illustrates a perspective view of a hybrid clip 100, also may be referred to as a hybrid safety spring clip, a hybrid needle guard, or a hybrid needle protective device, from the distal end of the hybrid clip. FIG. 20 illustrates a perspective view of the hybrid clip 100 from a proximal end. In the present embodiment, the hybrid clip 100 comprises a needle guard 132, which in an embodiment can have two resilient arms 288, 290, and two adaptors 350, each adaptor comprising an adaptor body 351, a biasing plate 353, and at least one engagement tab, which can extend in a distal direction or a proximal direction from the adaptor body.

In some embodiments, the partial circle of the adaptor body, such as the proximally facing surface of the adaptor body, can be used for bonding or affixing with the distally facing surface of the proximal wall 280 of the needle guard. Optionally, one or both adaptors can be co-molded or insert molded with the needle guard.

FIG. 19 illustrates an example (not claimed) that uses two adaptors 350, one for each of the two resilient arms 288, 290. As shown, each adaptor 350 has an adaptor body 351 that attaches to the proximal wall 280 and the biasing plate 353 extends over the outer surface of the respective arm 288, 290. The engagement tabs 358 extend over the side of the proximal wall 280 in the proximal direction.

The present example (not claimed) includes a center adaptor plate 390, configured to reinforce the opening 284 on the proximal wall 280 of the plastic needle guard 132. As shown, the center adaptor plate 390, which can be made from a metal material, is generally rectangular in shape and comprises a perimeter defining an opening 392. The opening 392 can be smaller than the largest dimension of a change in profile so that when used with a needle, proximal movement of the needle relative to the hybrid clip 100 will cause the change in profile to engage the opening 392. With further proximal movement and after the engagement, the change in profile can cause the hybrid clip 100 to then move in the proximal direction with the needle. The center adaptor plate 390 can attach to the proximal wall 280 via adhesive or bonding, or by insert or co-molding.

In an alternative example (not claimed), the three metal inserts of FIGs. 19 and 20, the two adaptors 350, and the center adaptor plate 390 can be formed as a one-piece structure and assembled or bonded to the needle guard 132 in a similar manner as the embodiment of FIGs. 10-16. Preferably the one-piece adaptor or the multi-piece adapters are only bonded or welded or insert molded to the proximal wall and not to the resilient arms. This allows the biasing plate 353 to flex independently of the resilient arm 288.

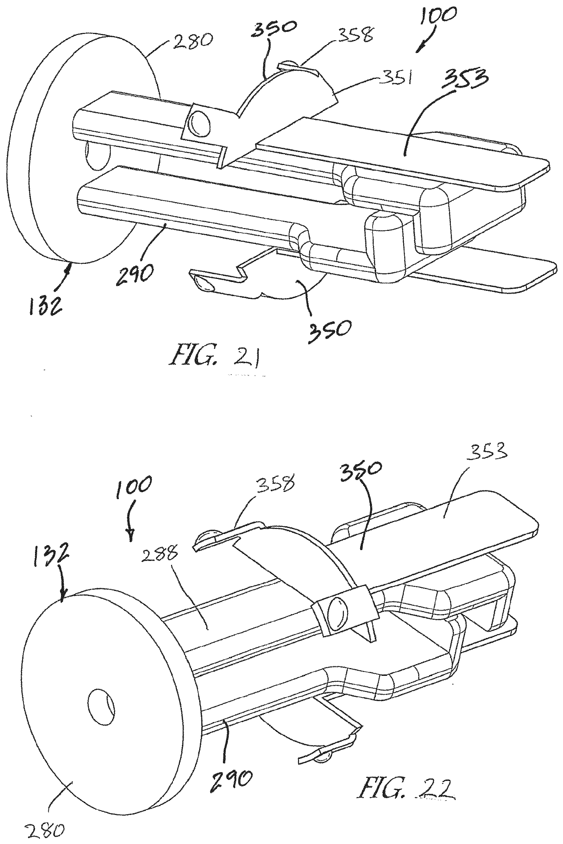

FIGs. 21 and 22 illustrate exploded views of the hybrid clip 100 of FIGs. 19 and 20, showing the needle guard 132 and the two adaptor 350 without the center adaptor plate 390.

Alternatively, FIGs. 21 and 22 can illustrate an example (not claimed) of a hybrid clip 100 having a needle guard 132 and two adaptors 350 where each adaptor 350 is located distal of the proximal wall 280 of the needle guard 132 and the adaptor body 351 is spaced apart from the proximal wall 280. The biasing plate 353 can be fixed to the respective resilient arm 288, 290 of the needle guard 132 by means of adhesive.

Again, the engagement tabs 358 may optionally be bent so that the free ends of each engagement tab extend in the distal direction or in the proximal direction.

In some embodiments, a hybrid clip 100 of the present invention can have at least one adaptor 350 with at least two engagement tabs 358, and wherein at least one of the engagement tabs 358 extends in the proximal direction and at least one of the engagement tabs 358 extends in the distal direction. A hybrid clip 100 (not claimed) can have two adaptors 350 with each adaptor 350 having at least one engagement tab 358, and wherein at least one of the engagement tabs 358 extends in the proximal direction and at least one engagement tab 358 extends in the distal direction.

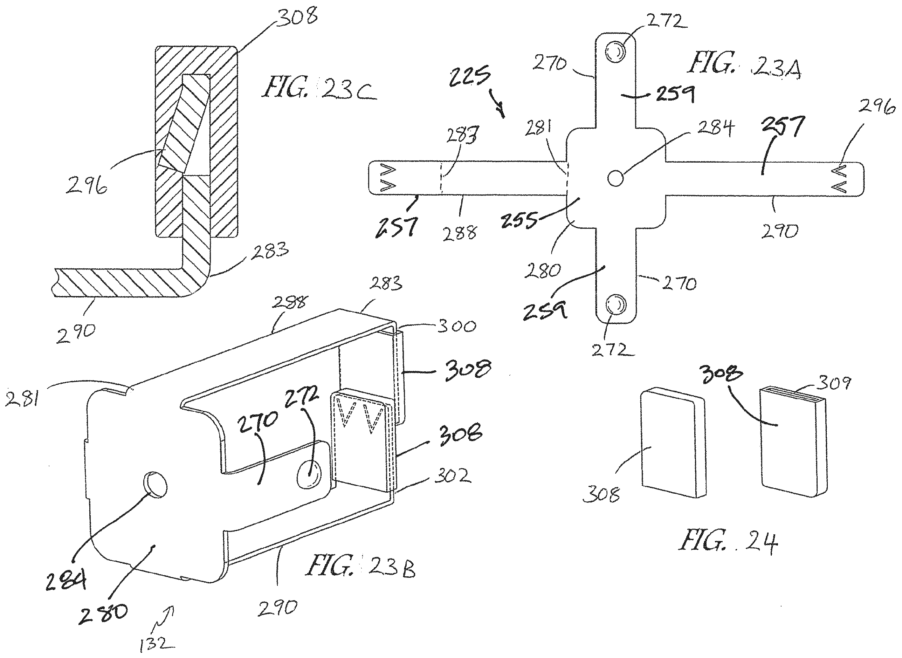

FIGs. 23A-23C and 24 are directed to a needle guard 132 formable from a single piece of material. Optionally, one or more wall guards 308, 308 made from a different material than the needle guard may be incorporated. The wall guards are configured to be placed on a wall or on wall sections of the needle guard. In instances where the wall guards are placed on the distal walls of the needle guard, the wall guards 308, 308 may be referred to as distal wall guards.

FIG. 23A illustrates a top plan view of a stamped material sheet 225 which can be bent to form a needle guard 132 (FIG. 23B). In some embodiments, the material can be stainless steel or other medical grade metallic materials in order to have good elastic properties. However, alternative materials can be considered, including plastics, such as engineered plastics like PEEK and PEK, and composites. The basic shape of a needle guard 132 stamped for bending into a needle guard can include a center portion 255, which can serve as a proximal wall 280 having an opening 284. In the embodiment of FIG. 23A, the center portion 225 that forms the proximal wall 280 has a generally rectangular shape with other shapes contemplated. On two opposed sides of the center portion can extend strips 257 of material corresponding to the resilient arms 288, 290. At end portions of the resilient arms 288, 290, at least one pointed tooth 296, or two pointed teeth 296, or other retention features, can be stamped or otherwise formed into the material. On the other opposed sides of the center portion, there can extend strips 259 of material corresponding to engagement tabs 270. Locating projections 272 can be formed on each strip 259 by coining or pressing a structural feature against an anvil.

Each of the strips 257, 257 on the stamped material sheet 225 can be bent in at least two locations to form the resilient arms 288, 290 and the engagement tabs 270. A first bend 281 can be performed near the center portion 255 for each longer strip 257. For the resilient arms 288, 290, a second bend 283 can be performed at an intermediary location along the strip 257 of material to form a distal wall. In this way, the resilient arms can be formed with distal walls 300 and 302 (FIG. 23B) pointing towards one another. Each of the second pair of strips 259, 259 can be bent at least one location 281 to form engagement tabs 270.

FIG. 24 illustrates distal wall guards 308 for use with the distal walls 300, 302. The distal wall guards 308 may be made from a soft plastic having a low coefficient of friction, such as low-density polyethylene (LDPE) or high-density polyethylene (HDPE). In use with a needle located between the two distal wall guards 308, the low coefficient of friction reduces the force necessary to withdraw the needle in the proximal direction relative to the two distal walls to allow the distal walls to block the needle tip from inadvertent needle sticks. The use of such low coefficient of friction guards can help with fixation of the needle after withdrawal by lessening sliding of the needle tip on the needle guard 132 compared to the needle tip contacting or hitting against a metallic surface. Instead, the needle tip can stick to the plastic wall guards 308 when in the protected position instead of sliding laterally when hitting and pivoting against bare metal. The plastic caps 308 can be extruded and cut to the width of the distal wall or molded, among other manufacturing methods. The distal wall guard 308 preferably has a slit 309 for fitment with a respective resilient arm, such as for receiving a distal wall of the resilient inside the space provided at the slit.

FIG. 23C illustrates how the distal wall guard 308 can mate with the teeth 296 of the resilient arm. With the stamped teeth being angled away from the end of the resilient arm and also set at an angle from the rest of the resilient arm 290, the teeth 296 can bite into the distal wall guard 308 like barbs once the distal wall guard 308 is put into place inside the slit. This arrangement of the teeth 296 allows for a light assembly force while also providing sufficient holding force so that the distal wall guard 308 does not come off during use. In some embodiments, the distal wall guard 308 can be installed prior to bending of the stamped metal sheet to form the needle guard 132.

FIG. 23B illustrates a needle guard 132 wherein the resilient arms 288, 290 and the engagement tabs 270 extend in a distal direction from the proximal wall 280. In some embodiments, the engagement tabs 270 can extend in the opposite proximal direction. However, it may be beneficial to have the engagement tabs 270 extend in the distal direction such that they cover the needle from sight and also provide a physical barrier to restrict the needle tip from sliding laterally out of the resilient arms.

In terms of retention of the needle guard 132 of FIG. 23B inside a catheter hub, it is envisioned that the proximal wall 280 of the needle guard 132 can be used to engage with a catheter hub, such as potentially by wedging or engaging the engagement tabs 270 with the interior of the catheter hub or against a Luer taper of the catheter hub. The proximal wall 280 has a proximally facing wall surface and a distally facing wall surface, similar to other proximal walls discussed elsewhere herein.

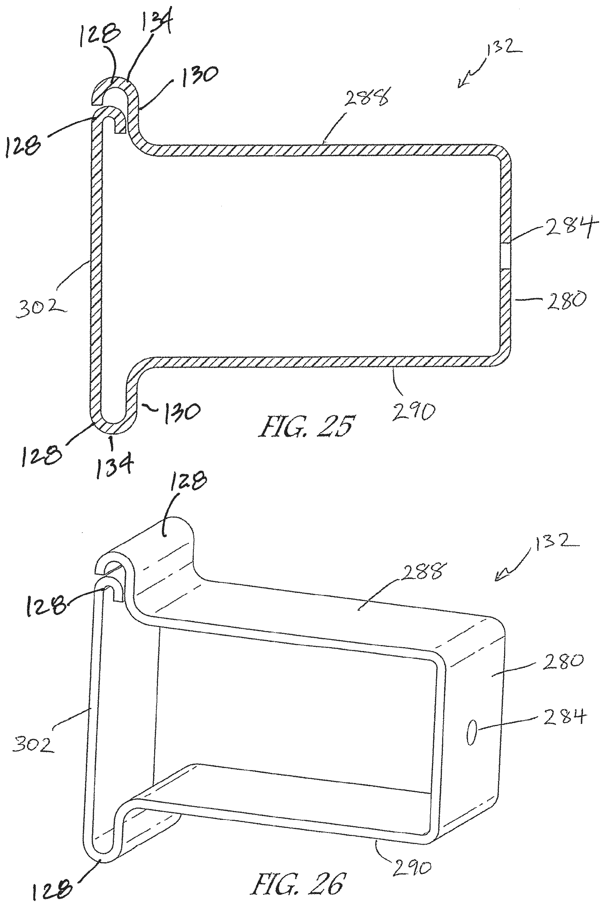

FIGs. 25-29 illustrate embodiments of needle guards utilizing one distal wall for blocking the needle tip, when in used with a needle or in a needle assembly, such as an IV catheter assembly.

FIG. 25 illustrates a cross-sectional side view of an embodiment of a needle guard 132 with two arms 288, 290 but only one distal wall 302 for blocking the needle tip. FIG. 26 is a perspective view from the proximal side of the needle guard 132 of FIG. 25. The needle guard 132 comprises a single strip of material stamped and bent to define a center portion or proximal wall 280 and the two arms. The proximal wall 280 can have a perimeter defining an opening 284 for a needle to pass therethrough. Extending distally from the proximal wall 280 are two resilient arms 288, 290. The two resilient arms 288, 290 can be substantially parallel to one another. Alternatively, the two resilient arms 288, 290 can be set an angle relative to the other.

At the distal portion of the first resilient arm 288, a curved protrusion or hook 128 can be incorporated. For example, the stamped metal sheet for forming the needle guard can be cold worked to form the curved protrusion or hook 128 at an end of the resilient arm 288. The curved protrusion 128 can include a first section 130 and a second section 134. The first section 130 can extend radially outwardly away from a lengthwise axis defined by the needle guard, and then terminated with a curved section to form the second section 134. The needle guard 132 may have more than one curved protrusion of the same size or of different sizes.

The second resilient arm 290 can incorporate a distal wall 302 extending at an angle to the lengthwise section of the arm, downstream of a curved protrusion 128, which has a first section 130 and second section 128. In some embodiments, the distal wall 302 can contact the curved protrusion 128 of the first resilient arm 288. As such, when the needle guard 132 of the present embodiment is in used and after a needle is withdrawn in a proximal direction relative to the needle guard following successful venipuncture, the single distal wall 302 can move back to cover the needle tip.

As shown, the second arm 290 has a second curved protrusion 128 at an end part of the distal wall 302. The second curved protrusion 128 can be smaller in size than the curved protrusion 128 on the first arm 288 so that when the two arms move closer together in the used position to cover the needle tip from inadvertent needle sticks, the smaller second curved protrusion 128 can recess within the larger profile of the larger curved protrusion 128 on the first arm 288. In other example, the two curved protrusions have approximately the same size so that the distal wall 302 is forced to deflect slightly outwardly in the distal direction when the needle guard is in the protected position shown in FIGs. 25 and 26.

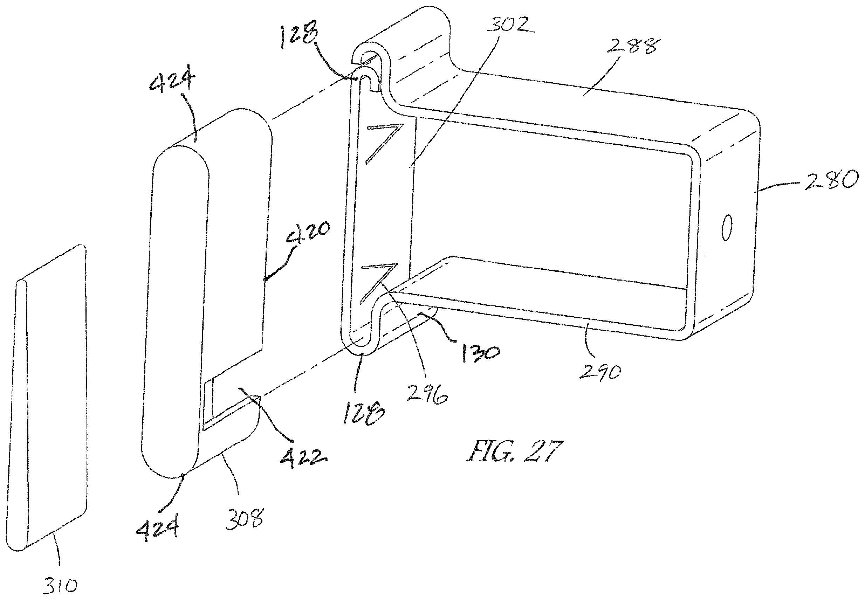

FIG. 27 illustrates a perspective view of the needle guard 132 of FIGs. 25 and 26 with two alternative distal wall guards 308, 310. In the first distal wall guard embodiment 308, a plastic cap having a wall structure with a lengthwise, lateral, opening 420 and a relief 422, which can have a three-sided cut-out, can be provided. The cap can have two ends 424 for receiving the two curved protrusions 128 on the second arm 290, one upstream of the distal wall and one downstream of the distal wall. The lateral opening 420 can slide laterally over the distal wall 302 and the curved protrusions 128. The arm section that extends from the first section 130 of the lower or upstream curved protrusion 128 can be accommodated by or can project out through the relief 422 formed with the distal wall guard 308.

The second distal wall guard embodiment 310 can embody a plate. In a particular example, the second distal wall guard embodiment can resemble a wedge or a wedge shape structure along a side cross-section. The distal wall guard 310 can be solid piece of plastic to be inserted on the proximal side of the distal wall 302 and held in place by fitment with the upstream and downstream curved protrusions 128 at the distal wall 302. In some embodiments, the plastic insert for the distal wall guard 310 can be taken from a roll, inserted into the clip from the side and then cut off from the rest of the roll. The distal wall guards may be held in place with contact with teeth 296.

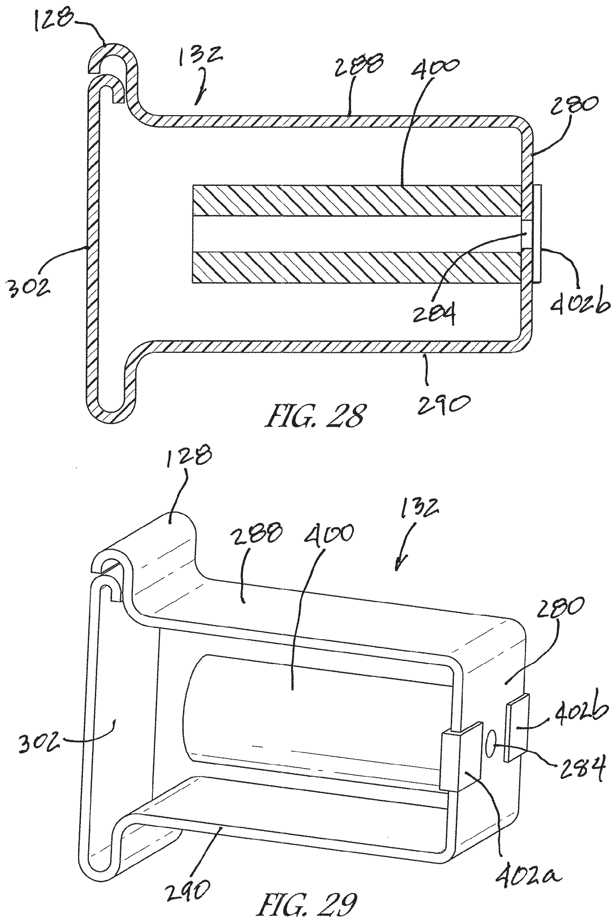

FIGs. 28 and 29 illustrate an embodiment of a needle guard 132 with an insert 400 secured to the proximal wall 280. FIG. 28 illustrates a cross-sectional view of the needle guard 132. FIG. 29 illustrates a perspective view of the needle guard 132 from the proximal end. In an example, the insert 400 can be made from a plastic material. The plastic insert 400 may be a tube secured to, attached to, or bonded to the proximal wall 280 and extending distally from the proximal wall, between the two arms 288, 290. The insert 400 can have a lumen or bore and the bore can be in communication or aligned with the through opening 284 of the proximal wall 280. In some embodiments, the insert 400 may be a cylinder with other outer surface geometries contemplated.

In some embodiments, the insert 400 may have clips 402a and 402b for clipping attachment to the needle guard 132. In an example, a stamped plate is formed with the insert 400, such as co-molded, insert molded, or bonded with the insert. The stamped plate is then folded around the proximal wall 280 to secure insert to the proximal wall. The stamped plate having the clips 402a, 402b can include a perimeter defining an opening for passing the needle therethrough. The opening on the stamped plate can have a smaller dimension than the largest dimension of the change in profile on the needle to enable the change in profile to engage the opening. The insert 400 is configured to guide the needle guard 132 and the needle during relative movement to limit or strict side-to-side or pivoting movement of the needle relative to the needle guard.

In some embodiments, the insert 400 can have a bore with a bore diameter that is slightly larger than the maximum diameter of the change in profile of the needle so that the crimp can be retracted to the metal proximal wall of the stamped plate to engage the opening. Alternatively, the bore of the plastic insert can also be smaller than the maximum diameter but slightly larger than a diameter of the needle shaft. In some embodiments of the latter case, the plastic insert can be placed on the proximal side of the proximal wall and it can be shorter as the clearance between the needle shaft and the guide bore can be kept to a minimum.

Methods of making and of using needle guards, adaptors for use with needle guards, needle assemblies and catheter assemblies for use with needle guards, including hybrid clips, of the present invention and components thereof as described herein are within the scope of the present disclosure. The methods are understood to include the use of a catheter assembly and withdrawing the needle such that surfaces of the needle guard are located to a side of the needle and then movable distal of the needle tip to cover the tip of the needle from inadvertent needle sticks.

Although limited embodiments of the needle guard, adaptor, needle assemblies and catheter assemblies and their applications have been specifically described and illustrated herein, including their components, many modifications and variations will be apparent to those skilled in the art. For example, the various features of the needle guards and the adaptors described herein may incorporate alternate materials, etc. Furthermore, it is understood and contemplated that features specifically discussed for one needle guard embodiment may be adopted for inclusion with another needle guard embodiment, provided the functions are compatible. Accordingly, it is to be understood that needle guards and their applications in catheter assemblies and needle assemblies constructed according to principles of the disclosed devices, systems, and methods may be embodied other than as specifically described herein. The invention is also defined in the following claims.

## Claims

1. A needle guard (132) for a sharp medical device, the needle guard (132) comprising:
a proximal wall (280) having a distally facing surface (287), a proximally facing surface (286), a side surface (285), and a perimeter defining a through opening (284);
a first arm (288) and a second arm (290) extending distally of the proximal wall (280); and
an adaptor (350) located between the first arm (288) and the second arm (290) and attached to the proximal wall (280), the adaptor (350) having a first engagement tab (358) and a second engagement tab (358) extending from an adaptor body (351),
**characterized by**
each of the first engagement tab (358) and the second engagement tab (358) comprising a locating protrusion (362) for contacting an interior surface of a hub or a housing.

2. The needle guard (132) according to claim 1, wherein the first engagement tab (358) extends from a side surface (356) of the adapter body (351) and comprises a bend (360) such that the first engagement tab (358) extends in a proximal or a distal direction from the bend (360).

3. The needle guard (132) according to claim 1, further comprising an insert (400) having a bore aligned with the through opening (284) of the proximal wall (280).

4. The needle guard (132) according to any one of the preceding claims, wherein
the proximal wall (280) is made of a first material;
the first arm (288) extends distally from the distally facing surface (287) of the proximal wall (280), the first arm (288) comprises an arm section and a distal wall (300);
the second arm (290) extends distally from the distally facing surface (287) of the proximal wall (280), the second arm (290) comprises an arm section and a distal wall (302);
wherein the first arm (288), the second arm (290), or both the first arm (288) and the second arm (290) are made of a second material different from the first material of the proximal wall (280).

5. The needle guard (132) according to claim 4, wherein the first arm (288) and the second arm (290) are made of the same second material.

6. The needle guard (132) according to any one of the preceding claims, wherein the locating protrusion (362) is used for contacting a catheter hub (106).

7. The needle guard (132) according to any one of the preceding claims, wherein the engagement tab (358) extends from the side surface (356) of the adaptor body (351) of the adapter (350) and comprises a bend (360) such that the engagement tab (358) extends in a proximal direction or a distal direction.

8. The needle guard (132) according to any one of the preceding claims, wherein the adaptor (350) comprises two or more engagement tabs (358).

9. The needle guard (132) according to claim 8, wherein at least two of the two or more engagement tabs (358) are diametrically opposed around an adaptor body (351).

## Patentansprüche

1. Nadelschutz (132) für eine scharfe medizinische Vorrichtung, wobei der Nadelschutz (132) umfasst:
eine proximale Wand (280) mit einer distal zugewandten Oberfläche (287), einer proximal zugewandten Oberfläche (286), einer Seitenoberfläche (285) und einem Umfang, der eine Durchgangsöffnung (284) definiert;
einen ersten Arm (288) und einen zweiten Arm (290), die sich distal von der proximalen Wand (280) erstrecken; und
einen Adapter (350), der zwischen dem ersten Arm (288) und dem zweiten Arm (290) angeordnet und an der proximalen Wand (280) befestigt ist, wobei der Adapter (350) eine erste Eingriffslasche (358) und eine zweite Eingriffslasche (358) aufweist, die sich von einem Adapterkörper (351) erstrecken,
**dadurch gekennzeichnet, dass**
die erste Eingriffslasche (358) sowie die zweite Eingriffslasche (358) jeweils einen Positionierungsvorsprung (362) zum Kontaktieren einer Innenoberfläche einer Nabe oder eines Gehäuses umfassen.

2. Nadelschutz (132) nach Anspruch 1, wobei sich die erste Eingriffslasche (358) von einer Seitenoberfläche (356) des Adapterkörpers (351) erstreckt und eine Biegung (360) umfasst, so dass sich die erste Eingriffslasche (358) von der Biegung (360) in eine proximale oder eine distale Richtung erstreckt.

3. Nadelschutz (132) nach Anspruch 1, ferner umfassend einen Einsatz (400) mit einer Bohrung, die an der Durchgangsöffnung (284) der proximalen Wand (280) ausgerichtet ist.

4. Nadelschutz (132) nach einem der vorhergehenden Ansprüche, wobei die proximale Wand (280) aus einem ersten Material hergestellt ist;
sich der erste Arm (288) distal von der distal zugewandten Oberfläche (287) der proximalen Wand (280) erstreckt und der erste Arm (288) einen Armabschnitt und eine distale Wand (300) umfasst;
sich der zweite Arm (290) distal von der distal zugewandten Oberfläche (287) der proximalen Wand (280) erstreckt und der zweite Arm (290) einen Armabschnitt und eine distale Wand (302) umfasst;
wobei der erste Arm (288), der zweite Arm (290) oder sowohl der erste Arm (288) als auch der zweite Arm (290) aus einem zweiten Material hergestellt sind, das sich vom ersten Material der proximalen Wand (280) unterscheidet.

5. Nadelschutz (132) nach Anspruch 4, wobei der erste Arm (288) und der zweite Arm (290) aus demselben zweiten Material hergestellt sind.

6. Nadelschutz (132) nach einem der vorhergehenden Ansprüche, wobei der Positionierungsvorsprung (362) zum Kontaktieren eines Katheteransatzes (106) verwendet wird.

7. Nadelschutz (132) nach einem der vorhergehenden Ansprüche, wobei sich die Eingriffslasche (358) von der Seitenoberfläche (356) des Adapterkörpers (351) des Adapters (350) erstreckt und eine Biegung (360) umfasst, so dass sich die Eingriffslasche (358) in eine proximale Richtung oder eine distale Richtung erstreckt.

8. Nadelschutz (132) nach einem der vorhergehenden Ansprüche, wobei der Adapter (350) zwei oder mehr Eingriffslaschen (358) umfasst.

9. Nadelschutz (132) nach Anspruch 8, wobei mindestens zwei der zwei oder mehr Eingriffslaschen (358) diametral gegenüberliegend um einen Adapterkörper (351) angeordnet sind.

## Revendications

1. Protège-aiguille (132) pour un dispositif médical pointu, le protège-aiguille (132) comprenant :
une paroi proximale (280) ayant une surface orientée de manière distale (287), une surface orientée de manière proximale (286), une surface latérale (285) et un périmètre définissant une ouverture traversante (284) ;
un premier bras (288) et un second bras (290) s'étendant de manière distale de la paroi proximale (280) ; et
un adaptateur (350) situé entre le premier bras (288) et le second bras (290) et attaché à la paroi proximale (280), l'adaptateur (350) ayant une première languette de mise en prise (358) et une seconde languette de mise en prise (358) s'étendant à partir d'un corps d'adaptateur (351),
**caractérisé par**
chacune de la première languette de mise en prise (358) et de la seconde languette de mise en prise (358) comprenant une saillie de positionnement (362) destinée à entrer en contact avec une surface intérieure d'un raccord ou d'un boîtier.

2. Protège-aiguille (132) selon la revendication 1, dans lequel la première languette de mise en prise (358) s'étend à partir d'une surface latérale (356) du corps d'adaptateur (351) et comprend une courbure (360) de telle sorte que la première languette de mise en prise (358) s'étend dans une direction proximale ou distale à partir de la courbure (360).

3. Protège-aiguille (132) selon la revendication 1, comprenant en outre un insert (400) ayant un alésage aligné sur l'ouverture traversante (284) de la paroi proximale (280).

4. Protège-aiguille (132) selon l'une quelconque des revendications précédentes, dans lequel la paroi proximale (280) est constituée d'un premier matériau ;
le premier bras (288) s'étend de manière distale à partir de la surface orientée de manière distale (287) de la paroi proximale (280), le premier bras (288) comprend une section de bras et une paroi distale (300) ;
le second bras (290) s'étend de manière distale à partir de la surface orientée de manière distale (287) de la paroi proximale (280), le second bras (290) comprend une section de bras et une paroi distale (302) ;
dans lequel le premier bras (288), le second bras (290), ou à la fois le premier bras (288) et le second bras (290) sont constitués d'un second matériau différent du premier matériau de la paroi proximale (280).

5. Protège-aiguille (132) selon la revendication 4, dans lequel le premier bras (288) et le second bras (290) sont constitués du même second matériau.

6. Protège-aiguille (132) selon l'une quelconque des revendications précédentes, dans lequel la saillie de positionnement (362) est utilisée pour entrer en contact avec un raccord de cathéter (106).

7. Protège-aiguille (132) selon l'une quelconque des revendications précédentes, dans lequel la languette de mise en prise (358) s'étend à partir de la surface latérale (356) du corps d'adaptateur (351) de l'adaptateur (350) et comprend une courbure (360) de telle sorte que la languette de mise en prise (358) s'étend dans une direction proximale ou une direction distale.

8. Protège-aiguille (132) selon l'une quelconque des revendications précédentes, dans lequel l'adaptateur (350) comprend deux languettes de mise en prise ou plus (358).

9. Protège-aiguille (132) selon la revendication 8, dans lequel au moins deux parmi les deux languettes de mise en prise ou plus (358) sont diamétralement opposées autour d'un corps d'adaptateur (351).
